# EUROPEAN PATENT APPLICATION

(11) **EP 4 582 456 A1**
(43) Date of publication of application: **09.07.2025**
(21) Application number: 23859423.8
(22) Date of filing: 30.08.2023
(51) Int. Cl.: C07K 19/00, C12N 5/10, C12N 15/10, A61K 35/17, A61K 39/395, A61P 35/00

(54) **STRENGTHENED RECEPTOR, AND IMMUNE CELL EXPRESSING STRENGTHENED RECEPTOR AND USE THEREOF**

(30) Priority: 30.08.2022 CN 202211057963
(71) Applicant: Chineo Medical Technology Co., Ltd., Beijing 102600 (CN)
(72) Inventor: ZHAO, Jie, Beijing 102600 (CN); GAO, Bin, Beijing 102600 (CN); GU, Weiyue, Beijing 102600 (CN)
(74) Representative: Winter, Brandl - Partnerschaft mbB
(86) International application number: PCT/CN2023/115934
(87) International publication number: WO 2024/046394

(57) **Abstract**

Provided are a TIGIT and OX40-based enhanced receptor (TIGIT/OX40) capable of increasing the activation level and survival time of immune cells, and an immune cell modified to express the enhanced receptor. Also provided is an immue cell modified to express two different enhanced receptors containing the enhanced receptor.

## Description

### FIELD OF THE INVENTION

The present invention belongs to the field of biomedical technology and specifically relates to the field of immune cell therapy.

### BACKGROUND OF THE INVENTION

A switch receptor is a fusion protein that has received much attention in the field of cell therapy in recent years. Sometimes called switch molecule, switch costimulatory receptor, or chimeric switch receptor, switch receptor is a transmembrane fusion protein, in which the extracellular segment is derived from a molecule capable of binding to a target cell and producing an immunosuppressive signal in a natural state, such as a checkpoint molecule, while the intracellular segment is derived from a molecule capable of producing an immunostimulatory signal, such as a costimulatory molecule. As a result, by modifying an immune cell with such a chimeric receptor, after the extracellular segment of the immunoreceptor binds to the target cell, the inhibitory signal that would otherwise be triggered is converted into a stimulatory signal by the chimeric receptor, thereby increasing the activity of the immune cell.

WO2013/019615 discloses a variety of chimeric switch receptors with extracellular segment derived from CTLA4, PD-1, or BTLA, and intracellular segment derived from CD28 or ICOS. Binding of CTLA4, PD-1 or BTLA to their natural ligands is supposed to generate a suppressive signal, but CD28 or ICOS in the intracellular domain of the chimeric switching receptor converts this suppressive signal into an activation signal. The application demonstrates that by expressing this chimeric switch receptor on T cells, the modified T cells exhibit a positive immune response, rather than the inhibitory response typically seen, when exposed to tumor cells expressing ligands for BTLA or PD-1.

As cell therapy and immunotherapy evolve, chimeric switch receptors with better efficacy are needed in this area.

### SUMMARY OF THE INVENTION

The inventors of the present invention have developed a new enhanced receptor that exhibits a significantly greater ability to activate immune cells as compared to chimeric switch receptors in the prior art, thereby completing the present invention.

Accordingly, in the first aspect, the present invention provides an enhanced receptor comprising an extracellular domain (ECD), a transmembrane domain (TM), and an intracellular domain (ICD), wherein the extracellular domain is derived from the extracellular domain of TIGIT, and the intracellular domain is derived from the intracellular domain of OX40.

In specific embodiments, the extracellular domain of TIGIT is the extracellular domain of human wild-type TIGIT, or a fragment or variant thereof that has a ligand binding capacity, wherein the ligand is CD155.

In the second aspect, the present invention provides an immune cell which is modified to express the enhanced receptors of the first aspect.

In the third aspect, the present invention provides an immune cell which is modified to express two different enhancement receptors, wherein the first enhancement receptor is the enhancement receptor of the first aspect of the present invention. Preferably, the second enhanced receptor has an extracellular domain which is capable of specifically binding to PD-L1 and the second enhanced receptor has an intracellular domain derived from CD28. Preferably, the extracellular segment of the second enhanced receptor is PD1 or a variant thereof that is capable of specifically binding to PD-L1 or is an anti-PD-L1 antibody.

In the fourth aspect, the present invention provides methods of preparing the immune cells of the second and third aspects.

In the fifth aspect, the present invention provides uses of the immune cells of the second and third aspects in the treatment of a disease.

In the sixth aspect, the present invention provides pharmaceutical compositions comprising the immune cells of the second aspect or the third aspect.

In the seventh aspect, the present invention provides nucleic acid molecules comprising a nucleotide sequence encoding an enhanced receptor of the first aspect. In further embodiments, the nucleic acid molecule further comprises a nucleotide sequence encoding a second enhanced receptor, the second enhanced receptor comprising a second extracellular domain, a second transmembrane domain, and a second intracellular domain,
wherein the second extracellular domain is capable of specifically binding to a ligand for a T cell immune checkpoint molecule different from TIGIT, wherein the second intracellular domain is derived from the co-stimulatory molecule that mediates immune cell activation signals.

In the eighth aspect, the present invention provides expression vectors that comprise the nucleic acid molecule of the seventh aspect.

In the ninth aspect, the present invention provides host cells that comprise the nucleic acid molecule of the seventh aspect, or the expression vectors of the eighth aspect.

### BRIEF DESCRIPTION OF THE FIGURES

FIGS. 1A-B are the results of in vitro experiments in Example 3 showing the secretion levels of IL-2 (A) and INFγ (B) from T cells after modification of tumor-recognizing T cells using different enhanced receptors.
FIG. 2 shows the results of the in vivo experiments in Example 4, showing the changes in tumor volume in the different treatment groups, illustrating the effect of the enhanced receptors on the tumor suppression effect.
FIG. 3 shows an enlarged view of the data of the remaining four groups of animals after removing the two controls (Freezing medium and Mock-T) in FIG. 2.
FIG. 4 shows the results of the in vitro experiments in Example 5, showing the secretion level of IL-2 from T cells after modification of tumor-recognizing T cells using different enhanced receptors.
FIG. 5 is the result of the in vitro experiment in Example 5, showing the secretion level of INFγ from T cells after modification of tumor-recognizing T cells using different enhanced receptors.
FIG. 6 shows the results of in vivo experiment in Example 5, showing the changes in tumor volume in the different treatment groups, illustrating the effect of the different enhanced receptors on the tumor suppression effect.
FIG. 7 shows an enlarged view of the data of the remaining four groups of animals after removing the control (Mock-T) in FIG. 6.
FIG. 8 shows the results of the in vivo experiment in Example 6, showing the changes in tumor volume in the different treatment groups.
FIG. 9 shows the changes in TCR copy number in peripheral blood of mice treated with different groups of therapeutic cells (TCR-T; PD1(108)/CD28-TCR-T; and PD1(108)/CD28-TIGIT/OX40-TCR-T) at different time points after injection over time.
FIG. 10 shows the changes in TCR copy number over time in tumor tissues of mice treated with different groups of therapeutic cells (TCR-T; PD1(108)/CD28-TCR-T; and PD1(108)/CD28-TIGIT/OX40-TCR-T) at different time points after injection.

### DETAILED DESCRIPTION OF THE INVENTION

### Definitions

The practice of some methods disclosed herein employ, unless otherwise indicated, conventional techniques of immunology, biochemistry, chemistry, molecular biology, microbiology, cell biology, genomics and recombinant DNA, which are within the skill of the art. See for example Sambrook and Green, Molecular Cloning: A Laboratory Manual, 4th Edition (2012); the series Current Protocols in Molecular Biology (F. M. Ausubel, et al. eds.); the series Methods In Enzymology (Academic Press, Inc.), PCR 2: A Practical Approach (M. J. MacPherson, B. D. Hames and G. R. Taylor eds. (1995)), Harlow and Lane, eds. (1988) Antibodies, A Laboratory Manual, and Culture of Animal Cells: A Manual of Basic Technique and Specialized Applications, 6th Edition (R. I. Freshney, ed. (2010)).

The term about or approximately means within an acceptable error range for the particular value as determined by one of ordinary skill in the art, which will depend in part on how the value is measured or determined, i.e., the limitations of the measurement system. Where specific values are described in the present application and claims, it should be assumed that the term "about" means within an acceptable margin of error of the specific value, unless otherwise stated.

The term enhanced receptor in the context herein refers to a recombinant transmembrane protein comprising an extracellular domain (ECD), a transmembrane domain (TM), and an intracellular domain (ICD), wherein the extracellular domain is derived from a protein of an immune cell and the protein triggers an immune cell deactivation signal upon binding to its ligand in unmodified immune cells, wherein the intracellular domain is derived from a co-stimulatory molecule mediating an immune cell activation signals. The enhanced receptors of the present invention can be used to modify immune cells. Immune cells modified to have the enhanced receptor generate immune cell activation signaling through binding of the extracellular domain of the enhanced receptor at the corresponding ligand, instead of immune cell inactivation signaling in the natural state upon binding of the extracellular domain to the corresponding ligand. In specific embodiments, the enhanced receptors of the present invention has an extracellular domain from TIGIT and an intracellular domain from OX40.

TIGIT means a T cell immunoreceptor with Ig and immunoreceptor tyrosine-based inhibitory domains.

OX40, also known as CD134, is a co-stimulatory immune checkpoint molecule belonging to the TNF receptor superfamily.

PD-1 refers to Programmed Death Receptor 1. PD-1 is an inhibitory immune checkpoint molecule expressed on the surface of B cells and T cells.

PD-L1 is one of the ligands for PD-1 and produces immunosuppressive signals after binding to PD-1.

CD28 is a receptor on the surface of T cells that provides a co-stimulatory signal for T cell activation.

The term nucleotide generally refers to a base-sugar-phosphate combination. A nucleotide may comprise a synthesized nucleotide. A nucleotide may comprise a synthesized nucleotide analog. A nucleotide may be a single unit of a nucleic acid sequence (for example, deoxyribonucleic acid (DNA) and ribonucleic acid (RNA)). The term nucleotide may comprise adenosine triphosphate (ATP), uridine triphosphate (UTP), cytosine triphosphate (CTP), guanosine triphosphate (GTP); and deoxyribonucleoside triphosphates, such as dATP, dCTP, dITP, dUTP, dGTP, dTTP; or derivatives thereof. Such derivatives may include, for example, [αS]dATP, 7-deaza-dGTP, and 7-deaza-dATP, as well as nucleotide derivatives that confer nuclease resistance to nucleic acid molecules containing them. As used herein, the term nucleotide may refer to dideoxyribonucleoside triphosphate (ddNTP) and derivatives thereof. Illustrative examples of dideoxyribonucleoside triphosphates may include, but are not limited to, ddATP, ddCTP, ddGTP, ddITP, and ddTTP. Nucleotides may be unlabeled, or may be detectedably labeled by well known techniques. Labeling can also be performed with quantum dots. Detectable labels may include, for example, radioisotopes, fluorescent labels, chemiluminescent labels, bioluminescent labels, and enzymatic labels.

The term gene refers to a nucleic acid (e.g., DNA such as genomic DNA and cDNA) and its corresponding nucleotide sequence that is involved in encoding an RNA transcript. The term as used herein with reference to genomic DNA includes intervening, non-coding regions as well as regulatory regions and can include 5' and 3' ends. In some uses, the term encompasses the transcribed sequences, including 5' and 3' untranslated regions (5' -UTR and 3' -UTR), exons and introns. In some genes, the transcribed region will contain open reading frames that encode polypeptides. In some uses of the term, a gene comprises only the coding sequences (e.g., an open reading frame or coding region ) necessary for encoding a polypeptide. In some cases, genes do not encode a polypeptide, for example, ribosomal RNA genes (rRNA) and transfer RNA (tRNA) genes. In some cases, the term gene includes not only the transcribed sequences, but in addition, also includes non-transcribed regions including upstream and downstream regulatory regions, enhancers and promoters. A gene can refer to an endogenous gene or a native gene in its natural location in the genome of an organism. A gene can refer to an exogenous gene or a non-native gene. A non-native gene can refer to a gene not normally found in the host organism but which is introduced into the host organism by gene transfer. A non-native gene can also refer to a gene not in its natural location in the genome of an organism. A non-native gene can also refer to a naturally occurring nucleic acid or polypeptide sequence that comprises mutations, insertions and/or deletions (e.g., non-native sequence).

The terms polynucleotide, oligonucleotide and nucleic acid are used interchangeably to refer to a polymerized form of nucleotides deoxyribonucleotides or ribonucleotides, or analogs thereof, of any length, whether in the form of a single-stranded, double-stranded, or multistranded . The polynucleotide may be exogenous or endogenous to the cell. The polynucleotide may be present in a cell-free environment. The polynucleotide may be a gene or a fragment thereof. The polynucleotide may be DNA. the polynucleotide may be RNA. The polynucleotide can have any three-dimensional structure and can perform any known or unknown function. The polynucleotide may comprise one or more analogs (for example, which have an altered backbone, sugar, or nucleobase).

The term expression refers to one or more processes by which a polynucleotide is transcribed from a DNA template (such as into an mRNA or other RNA transcript) and/or the process by which a transcribed mRNA is subsequently translated into peptides, polypeptides, or proteins. Transcripts and encoded polypeptides can be collectively referred to as gene product. If the polynucleotide is derived from genomic DNA, expression can include splicing of the mRNA in a eukaryotic cell. Up-regulated, with reference to expression, generally refers to an increased expression level of a polynucleotide (e.g., RNA such as mRNA) and/or polypeptide sequence relative to its expression level in a wild-type state while down-regulated generally refers to a decreased expression level of a polynucleotide (e.g., RNA such as mRNA) and/or polypeptide sequence relative to its expression in a wild-type state.

The term regulating with reference to expression or activity, as used herein, refers to altering the level of expression or activity. Regulation can occur at the transcription level and/or translation level.

The terms peptide, polypeptide, and protein are used interchangeably herein to refer to a polymer of at least two amino acid residues joined by peptide bond(s). This term does not connote a specific length of polymer, nor is it intended to imply or distinguish whether the peptide is produced using recombinant techniques, chemical or enzymatic synthesis, or is naturally occurring. The terms apply to naturally occurring amino acid polymers as well as amino acid polymers comprising at least one modified amino acid. In some cases, the polymer can be interrupted by non-amino acids. The terms include amino acid chains of any length, including full length proteins, and proteins with or without secondary and/or tertiary structure (e.g., domains). The terms also encompass an amino acid polymer that has been modified, for example, by disulfide bond formation, glycosylation, lipidation, acetylation, phosphorylation, oxidation, and any other manipulation such as conjugation with a labeling component.

The terms amino acid and amino acids, as used herein, generally refer to natural and non-natural amino acids, including, but not limited to, modified amino acids and amino acid analogues. Modified amino acids can include natural amino acids and non-natural amino acids, which have been chemically modified to include a group or a chemical moiety not naturally present on the amino acid. Amino acid analogues can refer to amino acid derivatives. The term amino acid includes both D-amino acids and L-amino acids.

The terms derivative, variant, and fragment, when used herein with reference to a polypeptide, refers to a polypeptide related to a wild type polypeptide, for example either by amino acid sequence, structure (e.g., secondary and/or tertiary), activity (e.g., enzymatic activity) and/or function. Derivatives, variants and fragments of a polypeptide can comprise one or more amino acid variations (e.g., mutations, insertions, and deletions), truncations, modifications, or combinations thereof compared to a wild type polypeptide.

As used herein, fusion can refer to a protein and/or nucleic acid comprising one or more non-native sequences (e.g., moieties). A fusion can comprise one or more of the same non-native sequences. A fusion can comprise one or more of different non-native sequences. A fusion can be a chimera. A fusion can comprise a nucleic acid affinity tag. A fusion can comprise a barcode. A fusion can comprise a peptide affinity tag. A fusion can provide for subcellular localization of the polypeptide (e.g., a nuclear localization signal (NLS) for targeting to the nucleus, a mitochondrial localization signal for targeting to the mitochondria, a chloroplast localization signal for targeting to a chloroplast, an endoplasmic reticulum (ER) retention signal, and the like). A fusion can provide a non-native sequence (e.g., affinity tag) that can be used to track or purify. A fusion can be a small molecule such as biotin or a dye such as Alexa fluor dyes, Cyanine3 dye, Cyanine5 dye.

The term neoantigen, as used herein, generally refers to tumor-specific antigens arising from mutations in a gene. The resulting mutated proteins, or fragments thereof, can trigger an antitumor T cell response.

The term genetic profile, as used herein, refers to information about specific genes, including variations and gene expression in an individual or in a certain type of tissue. A genetic profile can be used for neoantigen selection. The term somatic mutation profile, as used herein, refers to information about specific genes associated with somatic mutation, including but not limited to specific genes resulted from somatic mutation. A somatic mutation profile can be used for neoantigen selection.

The term antibody, as used herein, refers to a proteinaceous binding molecule with immunoglobulin-like functions. The term antibody includes antibodies (e.g., monoclonal and polyclonal antibodies), as well as derivatives, variants, and fragments thereof. Derivatives, variants, or fragments of antibody may refer to functional derivatives, or fragments that (for example, completely and/or partially) retain the binding specificity of the corresponding antibody. Antigen-binding fragments include Fab, Fab', F(ab')2 , variable fragment (Fv), single chain variable fragment (scFv), minibodies, diabodies, and single-domain antibodies (sdAb or nanobodies or camelids). The term antibody includes antibodies and antigen-binding fragments of antibodies that have been optimized, engineered or chemically conjugated. Examples of antibodies that have been optimized include affinity-matured antibodies. Examples of antibodies that have been engineered include Fc optimized antibodies (e.g., antibodies optimized in the fragment crystallizable region) and multispecific antibodies (e.g., bispecific antibodies).

The term artificial TCR, as used herein, is understood to mean an exogenous T cell receptor (TCR) complex, refers to a TCR complex in which one or more chains of the TCR are introduced into the genome of an immune cell that may or may not endogenously express the TCR. In some cases, an exogenous TCR complex can refer to a TCR complex in which one or more chains of an endogenous TCR complex have one or more mutated sequences, for example at either the nucleic acid or amino acid level. Expression of an exogenous TCR on an immune cell can confer binding specificity for an epitope or antigen (e.g., an epitope or antigen preferentially present on the surface of a cancer cell or other disease-causing cell or particle). An exogenous TCR complex can comprise a TCR-alpha, a TCR-beta chain, a CD3-gamma chain, a CD3-delta chain, a CD3-zeta chain, or any combination thereof, which is introduced into the genome. In some cases, the chain introduced into the genome may replace the endogenously occurring chain.

An immune checkpoint molecule is a molecule that regulates the immune response, usually a pair of receptor/ligand molecules that can have an inhibitory or stimulatory effect on the immune response.

The term Co-stimulatory molecule means, as used herein, a cell surface molecule that amplifies or counteracts the initial activation signal of a T cell. Unless otherwise specified, a co-stimulating molecule herein is a positive co-stimulating molecule, i.e., a co-stimulating molecule that generates an activation signal.

The terms subject, individual, and patient are used interchangeably herein to refer to a vertebrate, preferably a mammal such as a human. Mammals include, but are not limited to, murines, simians, humans, farm animals, sport animals, and pets. Tissues, cells and their progeny of a biological entity obtained in vivo or cultured in vitro are also encompassed.

The terms treatment and treating, as used herein, refer to an approach for obtaining beneficial or desired results including but not limited to a therapeutic benefit and/or a prophylactic benefit. For example, a treatment can comprise administering the cell, the cell population, or the composition disclosed herein. By therapeutic benefit is meant any therapeutically relevant improvement in or effect on one or more diseases, conditions, or symptoms under treatment. For preventive benefit, the cells, populations of cells, or the composition may be administered to a subject who is at risk of developing a particular disease, condition or symptom, or to a subject who was reported to have one or more physical symptoms of a disease, even though the disease, conditions or symptoms may not have have appeared yet.

The term effective amount or therapeutically effective amount refers to the quantity of a composition, for example a composition comprising immune cells such as lymphocytes (e.g., T lymphocytes) of the present disclosure, that is sufficient to result in a desired activity upon administration to a subject in need thereof. Within the context of the present disclosure, the term therapeutically effective refers to that quantity of a composition that is sufficient to delay the manifestation, arrest the progression, relieve or alleviate at least one symptom of a disorder treated by the present disclosure.

### TIGIT/OX40 enhanced receptor

TIGIT is a member of the poliovirus receptor (PVR)/nectin protein family, which is an immune receptor expressed predominantly on activated T cells, memory T cells, NK cells, and certain T regulatory cells (Tregs). TIGIT is able to bind to its high-affinity cognate ligand, CD155 (also known as the PVR), which is expressed on antigen-presenting cells (APCs), and this binding inhibits immune responses through T cells and APCs, and through NK cells. TIGIT has also been shown to compete with CD226 for binding to CD155, thereby balancing CD226-mediated co-stimulated T cells signaling. Because of these functions, TIGIT has become one of the popular targets in immunotherapy.

The TIGIT/OX40 enhanced receptor of the present invention comprises the extracellular domain of human TIGIT, or a fragment or variant thereof that has a ligand-binding capacity, wherein the ligand is CD155. Human TIGIT comprises 244 amino acids, wherein the extracellular domain comprises 141 amino acids and the transmembrane domain is 23 amino acids. In specific embodiments, the extracellular domain of the TIGIT/OX40 enhanced receptor of the present invention is the extracellular domain of human TIGIT, e.g., which has an amino acid sequence as shown in SEQ ID NO: 9.

The TIGIT/OX40 enhanced receptor of the present invention comprises the intracellular domain of human OX40, or a fragment or variant thereof having the function of forming a signaling complex. Human OX40 consists of 277 amino acids, including 37 amino acids at the cytoplasmic end, 217 amino acids in the extracellular domain, and 23 amino acids in the transmembrane domain. In specific embodiments, the intracellular domain of the TIGIT/OX40 enhanced receptor of the present invention is derived from the intracellular domain of wild-type human OX40, e.g., which has an amino acid sequence as shown in SEQ ID NO: 10.

In some embodiments, the enhanced receptor has TIGIT as its extracellular segment, when the intracellular segment is the intracellular domain derived from OX40, the enhanced receptor will enable T cells to secrete more cytokines, such as IL-2 and IFNy, and to survive longer in vivo, and longer survival in vivo. The enhanced receptor is therefore particularly preferred over the selection of intracellular domains derived from other co-stimulatory molecules, e.g., CD28, ICOS, and the like.

The transmembrane region of the TIGIT/OX40 enhanced receptor of the present invention may optionally be derived from TIGIT or OX40, such as comprising the transmembrane domain of TIGIT or OX40. In a preferred embodiment, the transmembrane domain is derived from OX40 and has an amino acid sequence as shown in SEQ ID NO: 11.

### The second enhanced receptor

Some embodiments of the present invention relate to an immune cell modified to express two different enhanced receptors (or also known as dual enhanced receptors), wherein the first enhanced receptor is a TIGIT/OX40 enhanced receptor of the present invention, and wherein the second enhanced receptor comprises a second extracellular domain, a second transmembrane domain, and a second intracellular domain, wherein the second extracellular domain is capable of specifically binding to a ligand for a T cell immune checkpoint molecule different from TIGIT, wherein the second intracellular domain is derived from the co-stimulatory molecule that mediates immune cell activation signals.

For the second extracellular domain is capable of specifically binding to a ligand for other T cell immune checkpoint molecule different from TIGIT, the other T cell immune checkpoint molecule may be selected from the group consisting of: transforming growth factor beta receptor (TGF-β-R), programmed cell death 1 (PD-1), cytotoxic T-lymphocyte-associated protein 4 (CTLA-4), B and T-lymphocyte attenuation factor (BTLA), killer cell immunoglobulin-like receptor (KIR), indoleamine 2,3-dioxygenase (IDO), lymphocyte activation gene 3 (LAG3) and T cell immunoglobulin mucin 3 (TIM-3). For example, the second extracellular domain may be an extracellular domain of other T cell immune checkpoint molecule different from TIGIT, or a fragment thereof that retains ligand-binding capacity. For example, the second extracellular domain may be an antibody or a fragment thereof, the antibody or fragment thereof specifically bind to ligands of other T cell immune checkpoint molecule different from TIGIT; for example, the antibody is a single chain antibody (scFv) or a single domain antibody (VHH).

In preferred embodiments, the other T cell immune checkpoint molecule is PD-1. Accordingly, the second extracellular domain may be PD-1 or a variant or fragment thereof that is capable of binding to PD-L1; or an anti-PD-L1 antibody or a fragment thereof that is capable of binding to PD-L1, such as a single-chain antibody (scFv) against PD-L1 or a single domain antibody (VHH) against PD-L1. In a specific embodiment, the extracellular domain of the second enhanced receptor has an amino acid sequence as shown in SEQ ID NO: 16. In a preferred embodiment, the extracellular domain of the second enhanced receptor is a variant of the extracellular domain of PD-1, and the variant has an improved affinity for PD-L1 relative to wild-type PD-1 and does not bind to at least one known PD-1 antibody, e.g., the extracellular domain of the second enhanced receptor has an amino acid sequence as shown in SEQ ID NO: 12.

In some embodiments, the co-stimulatory molecule from which the second intracellular domain is derived may be interleukin-2 receptor (IL-2R), interleukin-12 receptor (IL-12R), CD2, CD3, CD4, CD7, CD8, CD27, CD28, CD30, CD40, 4-1BB/CD137, ICOS, lymphocyte function-associated antigen-1 (LFA-1), LIGHT, NKG2C, or OX40. In some embodiments, the immune cell activation signal may be mediated by an activation factor. In some embodiments, the activation factor may be a soluble cytokine, a soluble chemokine, or a growth factor. In some embodiments, the activation factor may be a soluble cytokine, and wherein the soluble cytokine is IL-1, IL-2, IL-6, IL-7, IL-8, IL-10, IL-12, IL-15, IL-21, TNF, TGF, IFN, or any functional fragment or variant thereof. In some embodiments, the immune cell activation signal may comprise a clonal expansion of the modified immune cell; cytokine release by the modified immune cell; cytotoxicity of the modified immune cell; proliferation of the modified immune cell; differentiation, dedifferentiation or transdifferentiation of the modified immune cell; movement and/or trafficking of the modified immune cell; exhaustion and/or reactivation of the modified immune cell; and release of other intercellular molecules, metabolites, chemical compounds, or combinations thereof by the modified immune cell.

In a preferred embodiment, the co-stimulatory molecule is CD28. In a specific embodiment, the intracellular domain of the second enhanced receptor has an amino acid sequence as shown in SEQ ID NO: 13.

In a specific embodiment, the second enhanced receptor is a PD1/CD28 enhanced receptor comprising:
(a) a second extracellular domain, which is derived from PD-1 or a variant or fragment thereof that is capable of binding to PD-L1, such as an extracellular domain derived from PD-1 or a variant or fragment thereof that is capable of binding to PD-L1, preferably the second extracellular domain having an amino acid sequence as shown in SEQ ID NO: 16 or SEQ ID NO: 13;
(b) a second transmembrane domain, which is derived from PD-1 or CD28, for example , derived from the transmembrane domain of PD-1 or CD28, preferably a transmembrane domain derived from CD28, for example having an amino acid sequence as shown in SEQ ID NO: 14; and
(c) a second intracellular domain, which is derived from CD28, for example derived from the intracellular domain of CD28, for example having an amino acid sequence as shown in SEQ ID NO: 13.

In the case of having two different enhanced receptor modifications, the cells are able to convert immunosuppressive signals mediated by different immune checkpoint molecules into activation signals. In this way, immune cells with dual enhanced receptor modifications are able to exhibit further activation and immune response relative to immune cells with modifications with only one enhanced receptor. For example, the inventors have found that when immune cells with both TIGIT/OX40 enhanced receptor and PD-1(108)/CD28 enhanced receptor modifications are used, they are able to produce synergistic effect in suppressing tumors, the effect is significantly better than when immune cells modified with only one of the enhanced receptors alone are used. Accordingly, the present invention also relates to methods of enhancing the effects (e.g., immune activation and immune response effects) of therapeutic immune cells using the TIGIT/OX40 enhanced receptors of the present invention, wherein the therapeutic immune cells may have modifications other than the TIGIT/OX40 enhanced receptors, such as the second enhanced receptor and/or a chimeric antigen receptor of the present invention.

In embodiments comprising a second enhanced receptor, the nucleotide sequence encoding the TIGIT/OX40 enhanced receptor and the nucleotide sequence encoding the second enhanced receptor can be introduced into an immune cell for expression, either alone or in combination. For example, the nucleotide sequences encoding the two enhanced receptors can be linked with a self-cleaving peptide, such as a coding sequence of 2A peptides. The 2A peptides include, but are not limited to, T2A, P2A, E2A, F2A, and the like.

### Immune cells

The enhanced receptors of the present invention are used to modify immune cells, particularly lymphocytes such as T cells, in order to enhance the immune activation effect of the immune cells.

The immune cells, prior to being modified (in particular, prior to modification of the immune cells to express the enhanced receptors of the present invention), may be derived from a variety of sources, and prepared by any one or more of a variety of methods.

Immune cells that may be used in the present invention include: peripheral blood mononuclear cells(PBMCs), peripheral blood lymphocytes(PBL), and other blood cell subsets (e.g., but not limited to, T cells, natural killer cells, monocytes, natural killer T cells, monocyte precursor cells, hematopoietic stem cells, or non-pluripotent stem cells). In some embodiments, the cells may be any immune cells, including any T cell, such as a tumor infiltrating cell (TIL), CD3⁺ T cells, CD4⁺ T cells, or a CD8⁺ T cells. The T cell may also include a memory T cell, a memory stem T cell, or an effector T cell.

The immune cells can be a population of T cells, NK cells, B cells, and the like obtained from a subject. T cells can be obtained from a number of sources, including PBMCs, bone marrow, lymph node tissue, cord blood, thymus tissue, and tissue from a site of infection, ascites, pleural effusion, spleen tissue, and tumors. In some embodiments, T cells can be obtained from a unit of blood collected from a subject using any number of techniques, such as Ficoll^{™} separation. In one embodiment, cells from the circulating blood of an individual are obtained by apheresis. The apheresis product typically contains lymphocytes, including T cells, monocytes, granulocytes, B cells, other nucleated white blood cells, red blood cells, and platelets. The cells collected by apheresis may be washed to remove the plasma fraction and to place the cells in an appropriate buffer or media for subsequent processing steps.

The T cells can also be selected from a bulk population, for example, selecting T cells from whole blood. The T cells can also be expanded from a bulk population. During selection and expansion, the T cells can be skewed towards particular populations and phenotypes. For example, the T cells can be skewed to phenotypically comprise, CD45RO(-), CCR7(+), CD45RA(+), CD62L(+), CD27(+), CD28(+) and/or IL-7Ra(+). Suitable cells can be selected that comprise one of more markers selected from a list comprising: CD45RO(-), CCR7 (+), CD45RA (+), CD62L (+), CD27 (+), CD28 (+) and/or IL-7Ra (+).

Stem cells can also be used to obtain immune cells through differentiation. The stem cells are such as, embryonic stem cells, induced pluripotent stem cells, hematopoietic stem cells, neuronal stem cells and mesenchymal stem cells. The immune cells can comprise any number of primary cells, such as human cells, non-human cells, and/or mouse cells.

The immune cells can be derived from the subject to be treated (e.g., patient). The immune cells can be derived from a healthy human donor. The immune cells may be derived from a donor that has direct relationship with the subject, such as a donor that is HLA hemi-compatible with the subject.

Preferably, the immune cells have recognition specificity for a target cell, such as a tumor cell. This conferral of recognition specificity may be achieved by various methods including, but not limited to, genetic modification, by co-incubation with antigen-presenting cells loaded with the target cells antigens, and/or by selecting specific cell populations.

In one embodiment, the immune cells of the present invention may be tumor infiltrating lymphocytes (TIL). In some embodiments, the TIL may express at least one of PD-1, CD137, and TIM-3.

In some embodiments, the T cells can comprise a T cell receptor (TCR) complex exhibiting specific binding to a neoantigen. In some embodiments, the TCR complex may be an endogenous TCR complex. In some embodiments, the TCR complex may be an exogenous TCR complex.

In some embodiments, the neoantigen may be selected based on a genetic profile of a tumor sample from an individual. In some embodiments, the neoantigen may be selected based on a somatic mutation profile of a tumor sample from an individual.

In a particular embodiment, the immune cells of the present invention are derived from peripheral blood mononuclear cells (PBMCs) and are sorted to enrich the PBMC for PD1⁺ T cells. "Enrichment" implies a treatment that results in an increase in the number or percentage of a certain cell or component in a population. Since in practical laboratory and clinical operations, the process of cell preparation, sorting, or modification is often performed on samples containing multiple cells(cell populations). Such an enrichment operation implies that, prior to being modified to express the enhanced receptors, the immune cells or immune cell populations preferably used for further modifications in the present invention should contain a higher proportion of PD1⁺ T cells, than the PBMC populations not subjected to the enrichment. For example, for patients with advanced solid tumors, the percentage of peripheral blood PD1⁺ T cells averages around 28%. When immune cells are offered for modification as the immune cells of the present invention, this percentage is preferably further increased by enrichment, e.g., sorting processes. In specific embodiments, the percentage of PD1⁺ cells in the cell population derived from peripheral blood PBMC and undergone positive selection for PD-1 is greater than 50%, preferably greater than 60%, more preferably greater than 70%, and particularly preferably greater than 80%.

After modification, any of the immune cells obtained through the methods and sources described above-particularly those modified to express the enhanced receptors of the present invention-are referred to as modified immune cells.

### Other modifications

The modified immune cells of the present invention may have one or more other modifications in addition to expressing the enhanced receptor to confer further functions and/or properties.

The immune cells of the present invention may be modified to express a chimeric antigen receptor (CAR), the chimeric antigen receptor comprising (i) an antigen-interacting domain that can bind to a B cell surface protein; (ii) a transmembrane domain; and (iii) an intracellular signaling domain. An immune cell with the CAR is capable of achieving expansion and/or activation of the immune cell after contact with a B cell surface protein.

In some embodiments, the B cell surface protein can be any protein that may be found on the surface of a B cell. Non-limiting examples include CD1d, CD5, CD10, CD11a, CD19, CD20, CD21, CD22, CD23, CD24, CD25, CD27, CD28, CD29, CD34, CD37, CD38, CD40, CD44, CD45, CD49b, CD69, CD72, CD74, CD80, CD83, CD84, CD86, CD93, CD95, CD117, CD127, CD138, CD147, CD148, CD185, CD270, CD284 and CD360. In some embodiments, the antigen-interacting domain of the CAR is capable of binding to a surface protein on a non-B cell, provided that this binding to the surface protein does not significantly compromise the overall state of health or immune system of the host. In some embodiments, the surface protein is a surface protein on an immune cell. In some embodiments, the surface protein is a surface protein on a cell other than an immune cell. In some embodiments, the surface protein may be selected from, with the proviso that the binding to the surface protein does not significantly compromise the general health status or the immune system of the host, CD31, CD32A, CD32B, CD33, CD34, CD35, CD36, CD37, CD38, CD39, CD40, CD41, CD42a, CD42b, CD42c, CD42d, CD43, CD44, CD45, CD46, CD47, CD48, CD49(a, b, c, d, e, f), CD50, CD51, CD52, CD53, CD54, CD55, CD56, CD57, CD58, CD59, CD61, CD62(E, L, P), CD63, CD64(A, B, C), CD66(a, b, c, d, e, f), CD68, CD69, CD70, CD71, CD72, CD73, CD74, CD78, CD79(a, b), CD80, CD81, CD82, CD83, CD84, CD85(a, d, e, h, j, k), CD86, CD87, CD88, CD89, CD90, CD91, CD92, CD93, CD94, CD95, CD96, CD97, CD98, CD99, CD100, CD1(a-c), 1A, 1D, 1E, CD2, CD3(y, δ, ε), CD4, CD5, CD6, CD7, CD8, a, CD9, CD10, CD11(a, b, c, d), CD13, CD14, CD15, CD16A, CD16B, CD18, CD19, CD20, CD21, CD22, CD23, CD24, CD25, CD26, CD27, CD28, CD29, CD30, CD101, CD102, CD103, CD104, CD105, CD106, CD107(a, b), CD108, CD109, CD110, CD111, CD112, CD113, CD114, CD115, CD116, CD117, CD118, CD119, CD120(a, b), CD121(a, b), CD122, CD123, CD124, CD125, CD126, CD127, CD129, CD130, CD131, CD132, CD133, CD134, CD135, CD136, CD137, CD138, CD140b, CD141, CD142, CD143, CD144, CD146, CD147, CD148, CD150, CD191, CD192, CD193, CD194, CD195, CD196, CD197, CDw198, CDw199, CD200, CD201, CD202b, CD204, CD205, CD206, CD207, CD208, CD209, CDw210(a, b), CD212, CD213a(1, 2), CD217, CD218(a, b), CD220, CD221, CD222, CD223, CD224, CD225, CD226, CD227, CD228, CD229, CD230, CD233, CD234, CD235(a, b), CD236, CD238, CD239, CD240CE, CD240D, CD241, CD243, CD244, CD246, CD247, CD248, CD249, CD252, CD253, CD254, CD256, CD257, CD258, CD261, CD262, CD263, CD264, CD265, CD266, CD267, CD268, CD269, CD271, CD272, CD273, CD274, CD275, CD276, CD278, CD279, CD280, CD281, CD282, CD283, CD284, CD286, CD288, CD289, CD290, CD292, CDw293, CD294, CD295, CD297, CD298, CD299, CD300A, CD301, CD302, CD303, CD304, CD305, CD306, CD307, CD309, CD312, CD314, CD315, CD316, CD317, CD318, CD320, CD321, CD322, CD324, CD325, CD326, CD328, CD329, CD331, CD332, CD333, CD334, CD335, CD336, CD337, CD338, CD339, CD340, CD344, CD349, CD350, CD151, CD152, CD153, CD154, CD155, CD156(a, b, c), CD157, CD158(a, d, e, i, k), CD159(a, c), CD160, CD161, CD162, CD163, CD164, CD166, CD167(a, b), CD168, CD169, CD170, CD171, CD172(a, b, g), CD174, CD177, CD178, CD179(a, b), CD180, CD181, CD182, CD183, CD184, CD185 and CD186. In a preferred embodiment, the B cell surface protein is selected from CD19, CD20, and CD22.

In some embodiments, the antigen-interacting domain of the CAR can be a B cell surface protein or a fragment thereof that is capable of binding to a dead B cell. B cell apoptosis could occur before or after development of an immune response (e.g., an immune response towards a tumor cell). Thus, a dead B cell or its debris can still have a B cell surface protein or the fragment thereof presented on the surface. The ability of the CAR to target both live and dead B cells may increase the chance of the immune cell comprising the CAR to (i) bind to the B cell surface protein and (i) initiate the signaling of the intracellular signaling domain. In some cases, the signaling of the intracellular signaling domain may promote expansion (proliferation) of the immune cell comprising the CAR.

In some embodiments, the antigen interacting domain of the CAR is capable of binding to a B cell surface protein or a fragment thereof that is coupled (e.g., via a covalent and/or non-covalent bond) to a surface of particles (e.g., nanoparticles). The particles may be any particulate materials comprising organic and/or inorganic material. The particles may have various shapes and sizes. The particles may be about 1 nanometers (nm) to about 50 micometers (µm) in at least one dimension. The particles may be at least about 1 nm, 5 nm, 10 nm, 50 nm, 100 nm, 500 nm, 1 µm, 5 µm, 10 µm, 50 µm, or more in at least one dimension. The particles may be at most about 50 µm, 10 µm, 5 µm, 1 µm, 500 nm, 100 nm, 50 nm, 10 nm, 5 nm, 1 nm, or less in at least one dimension. The particles may be nanoparticles, microparticles, nanospheres, micropsheres, nanorods, microrods, nanofibers, nanoribbons, etc. Examples of the particle include metal nanoparticles (e.g., gold nanoparticles, silver nanoparticles, and iron nanoparticles), intermetallic nanosemiconductor nanoparticles, core-shell nanoparticles, particles with an inorganic core with a polymer shell, particles with an organic core with a polymer shell, and mixtures thereof. Alternatively, the particles can be organic nanoparticles, such as crosslinked polymers, hydrogel polymers, biodegradable polymers, polylactide (PLA), polyglycolide (PGA), polycaprolactone (PCL), copolymers, polysaccharides, starch, cellulose, chitosan, polyhydroxyalkanoates (PHA), PHB, PHV, lipids, peptides, peptide amphiphiles, polypeptides (e.g., proteins), or combinations thereof. The particles presenting the B cell surface protein on the surface may be introduced in vitro to the immune cell comprising the CAR that binds the B cell surface protein. Alternatively or in addition to, the particles presenting the B cell surface protein may be introduced in vivo (e.g., local or systemic injection) along with the immune cell comprising the CAR. Such particles may be used to expand the population of the immune cell comprising the CAR in vitro or in vivo.

The antigen binding domain can comprise any protein or molecule capable of binding to an antigen, e.g., B cell surface protein. Non-limiting examples of the antigen binding domain include, but are not limited to, a monoclonal antibody, a polyclonal antibody, a recombinant antibody, a human antibody, a humanized antibody, a murine antibody, or a functional derivative, variant or fragment thereof, including, but not limited to, a Fab, a Fab', a F(ab')2, an Fv, a single-chain Fv (scFv), minibody, a diabody, and a single-domain antibody such as a heavy chain variable domain (VH), a light chain variable domain (VL) and a variable domain (VHH) of camelid derived nanobody. In some embodiments, the first antigen binding domain comprises at least one of a Fab, a Fab', a F(ab')2, an Fv, and a scFv. In some embodiments, the antigen binding domain comprises an antibody mimetic. Antibody mimetics refer to molecules which can bind a target molecule with an affinity comparable to an antibody, and include single-chain binding molecules, cytochrome b562-based binding molecules, fibronectin or fibronectin-like protein scaffolds (e.g., adnectins), lipocalin scaffolds, calixarene scaffolds, A-domains and other scaffolds. In some embodiments, an antigen binding domain comprises a transmembrane receptor, or any derivative, variant, or fragment thereof. For example, an antigen binding domain can comprise at least a ligand binding domain of a transmembranereceptor.

In some embodiments, the antigen binding domain can comprise a scFV. A scFv can be derived from an antibody for which the sequences of the variable regions are known. In some embodiments, a scFv can be derived from an antibody sequence obtained from an available mouse hybridoma. A scFv can be obtained from whole-exomic sequencing of a tumor cell or primary cell. In some embodiments, a scFv can be altered. For instance, a scFv may be modified in a variety of ways. In some cases, a scFv can be mutated, so that the scFv may have higher affinity to its target. In some cases, the affinity of the scFv for its target can be optimized for targets expressed at low levels on normal tissues. This optimization can be performed to minimize potential toxicities, such as hypercytokinemia. In other cases, the cloning of a scFv that has a higher affinity for the membrane bound form of a target can be preferable over its soluble form counterpart. This modification can be performed if some targets can also be detected in soluble form at different levels and their targeting can cause unintended toxicity, such as hypercytokinemia.

The antigen binding domain of a CAR of a subject system can be linked to the intracellular signaling domain via a transmembrane domain. A transmembrane domain of a subject CAR can anchor the CAR to the plasma membrane of a cell, for example an immune cell. In some embodiments, the membrane spanning segment comprises a polypeptide. The membrane spanning polypeptide linking the antigen binding domain and the intracellular signaling domain of the CAR can have any suitable polypeptide sequence. In some cases, the membrane spanning polypeptide comprises a polypeptide sequence of a membrane spanning portion of an endogenous or wild-type membrane spanning protein. In some embodiments, the membrane spanning polypeptide comprises a polypeptide sequence having at least 1 (e.g., at least 2, 3, 4, 5, 6, 7, 8, 9, 10 or greater) of an amino acid substitution, deletion, and insertion compared to a membrane spanning portion of an endogenous or wild-type membrane spanning protein. In some embodiments, the membrane spanning polypeptide comprises a non-natural polypeptide sequence, such as the sequence of a polypeptide linker. The polypeptide linker may be flexible or rigid. The polypeptide linker can be structured or unstructured. In some embodiments, the membrane spanning polypeptide transmits a signal from an extracellular region of a cell to an intracellular region, for via the antigen binding domain. A native transmembrane portion of CD28 can be used in a CAR. In other cases, a native transmembrane portion of CD8 alpha can also be used in a CAR.

A CAR of the present disclosure can comprise a signaling domain, or any derivative, variant, or fragment thereof, involved in immune cell signaling. The intracellular signaling domain of a CAR can induce activity of an immune cell comprising the CAR. The intracellular signaling domain can transduce the effector function signal and direct the cell to perform a specialized function. The signaling domain can comprise signaling domains of other molecules. In some cases, a truncated portion of the signaling domain is used in a CAR.

In some embodiments, the intracellular signaling domain comprises multiple signaling domains involved in immune cell signaling, or any derivatives, variants, or fragments thereof. For example, the intracellular signaling domain can comprise at least 2 immune cell signaling domains, e.g., at least 2, 3, 4, 5, 7, 8, 9, or 10 immune cell signaling domains. An immune cell signaling domain can be involved in regulating primary activation of the TCR complex in either a stimulatory way or an inhibitory way. The intracellular signaling domain may be that of a T-cell receptor (TCR) complex. The intracellular signaling domain of the CAR of the invention can comprise a signaling domain of an Fcγ receptor (FcγR), an Fcε receptor (FcεR), an Fcα receptor (FcαR), neonatal Fc receptor (FcRn), CD3, CD3ζ, CD3γ, CD3δ, CD3ε, CD4, CD5, CD8, CD21, CD22, CD28, CD32, CD40L (CD154), CD45, CD66d, CD79a, CD79b, CD80, CD86, CD278 (also known as ICOS), CD247 ζ, CD247 η, DAP10, DAP12, FYN, LAT, Lck, MAPK, MHC complex, NFAT, NF-κB, PLC-y, iC3b, C3dg, C3d, and Zap70. In some embodiments, the signaling domain includes an immunoreceptor tyrosine-based activation motif or ITAM. A signaling domain comprising an ITAM can comprise two repeats of the amino acid sequence YxxL/I separated by 6-8 amino acids, wherein each x is independently any amino acid, producing the conserved motif YxxL/Ix(6-8)YxxL/I. A signaling domain comprising an ITAM can be modified, for example, by phosphorylation when the antigen binding domain is bound to an epitope. A phosphorylated ITAM can function as a docking site for other proteins, for example proteins involved in various signaling pathways. In some embodiments, the primary signaling domain comprises a modified ITAM domain, e.g., a mutated, truncated, and/or optimized ITAM domain, which has altered (e.g., increased or decreased) activity compared to the native ITAM domain.

In some embodiments, the intracellular signaling domain of the CAR of present invention comprises an FcyR signaling domain (e.g., ITAM). The FcyR signaling domain can be selected from FcγRI (CD64), FcyRIIA (CD32), Fc γRIIB (CD32), FcγRIIIA (CD16a), and FcγRIIIB (CD16b). In some embodiments, the intracellular signaling domain comprises an FcεR signaling domain (e.g., ITAM). The FcεR signaling domain can be selected from FcεRI and FcεRII (CD23). In some embodiments, the intracellular signaling domain comprises an Fc R signaling domain (e.g., ITAM). The Fc αR signaling domain can be selected from FcαRI (CD89) and Fcα/µR. In some embodiments, the intracellular signaling domain comprises a CD3ζ signaling domain. In some embodiments, the primary signaling domain comprises an ITAM of CD3ζ.

In some embodiments, an intracellular signaling domain of a subject CAR comprises an immunoreceptor tyrosine-based inhibition motif or ITIM. A signaling domain comprising an ITIM can comprise a conserved sequence of amino acids (S/I/V/LxYxxIV/L) that is found in the cytoplasmic tails of some inhibitory receptors of the immune system. A primary signaling domain comprising an ITIM can be modified, for example phosphorylated, by enzymes such as a Src kinase family member (e.g., Lck). Following phosphorylation, other proteins, including enzymes, can be recruited to the ITIM. These other proteins include, but are not limited to, enzymes such as the phosphotyrosine phosphatases SUP-1 and SHP-2, the inositol-phosphatase called SHIP, and proteins having one or more SH2 domains (e.g., ZAP70). A intracellular signaling domain can comprise a signaling domain (e.g., ITIM) of BTLA, CD5, CD31, CD66a, CD72, CMRF35H, DCIR, EPO-R, Fc yRIIB (CD32), Fc receptor-like protein 2 (FCRL2), Fc receptor-like protein 3 (FCRL3), Fc receptor-like protein 4 (FCRL4), Fc receptor-like protein 5 (FCRL5), Fc receptor-like protein 6 (FCRL6), protein G6b (G6B), interleukin 4 receptor (IL4R), immunoglobulin superfamily receptor translocation-associated 1 (IRTA1), immunoglobulin superfamily receptor translocation-associated 2 (IRTA2), killer cell immunoglobulin-like receptor 2DL1 (KIR2DL1), killer cell immunoglobulin-like receptor 2DL2 (KIR2DL2), killer cell immunoglobulin-like receptor 2DL3 (KIR2DL3), killer cell immunoglobulin-like receptor 2DL4 (KIR2DL4), killer cell immunoglobulin-like receptor 2DL5 (KIR2DL5), killer cell immunoglobulin-like receptor 3DL1 (KIR3DL1), killer cell immunoglobulin-like receptor 3DL2 (KIR3DL2), leukocyte immunoglobulin-like receptor subfamily B member 1 (LIR1), leukocyte immunoglobulin-like receptor subfamily B member 2 (LIR2), leukocyte immunoglobulin-like receptor subfamily B member 3 (LIR3), leukocyte immunoglobulin-like receptor subfamily B member 5 (LIR5), leukocyte immunoglobulin-like receptor subfamily B member 8 (LIR8), leukocyte-associated immunoglobulin-like receptor 1 (LAIR-1), mast cell function-associated antigen (MAFA), NKG2A, natural cytotoxicity triggering receptor 2 (NKp44), NTB-A, programmed cell death protein 1 (PD-1), PTLR, SIGLECL1, sialic acid binding Ig like lectin 2 (SIGLEC2 or CD22), sialic acid binding Ig like lectin 3 (SIGLEC3 or CD33), sialic acid binding Ig like lectin 5 (SIGLEC5 or CD170), sialic acid binding Ig like lectin 6 (SIGLEC6), sialic acid binding Ig like lectin 7 (SIGLEC7), sialic acid binding Ig like lectin 10 (SIGLEC10), sialic acid binding Ig like lectin 11 (SIGLEC11), sialic acid binding Ig like lectin 4 (SIGLEC4), sialic acid binding Ig like lectin 8 (SIGLEC8), sialic acid binding Ig like lectin 9 (SIGLEC9), platelet and endothelial cell adhesion molecule 1 (PECAM-1), signal regulatory protein (SIRPα2), and signaling threshold regulating transmembrane adaptor 1 (SIT). In some embodiments, the intracellular signaling domain comprises a modified ITIM domain, e.g., a mutated, truncated, and/or optimized ITIM domain, which has altered (e.g., increased or decreased) activity compared to the native ITIM domain.

In some embodiments, the intracellular signaling domain comprises at least 2 ITAM domains (e.g., at least 3, 4, 5, 6, 7, 8, 9, or 10 ITAM domains). In some embodiments, the intracellular signaling domain comprises at least 2 ITIM domains (e.g., at least 3, 4, 5, 6, 7, 8, 9, or 10 ITIM domains) (e.g., at least 2 primary signaling domains). In some embodiments, the intracellular signaling domain comprises both ITAM and ITIM domains.

In some cases, the intracellular signaling domain of a subject CAR can include a co-stimulatory domain. In some embodiments, a co-stimulatory domain, for example from co-stimulatory molecule, can provide co-stimulatory signals for immune cell signaling, such as signaling from ITAM and/or ITIM domains, e.g., for the activation and/or deactivation of immune cell activity. In some embodiments, a co-stimulatory domain is operable to regulate a proliferative and/or survival signal in the immune cell. In some embodiments, a co-stimulatory signaling domain comprises a signaling domain of a MHC class I protein, MHC class II protein, TNF receptor protein, immunoglobulin-like protein, cytokine receptor, integrin, signaling lymphocytic activation molecule (SLAM protein), activating NK cell receptor, BTLA, or a Toll ligand receptor. In some embodiments, the co-stimulatory domain comprises a signaling domain of a molecule selected from the group consisting of: 2B4/CD244/SLAMF4, 4-1BB/TNFSF9/CD137, B7-1/CD80, B7-2/CD86, B7-H1/PD-L1, B7-H2, B7-H3, B7-H4, B7-H6, B7-H7, BAFF R/TNFRSF13C, BAFF/BLyS/TNFSF13B, BLAME/SLAMF8, BTLA/CD272, CD100(SEMA4D), CD103, CD11a, CD11b, CD11c, CD11d, CD150, CD160(BY55), CD18, CD19, CD2, CD200, CD229/SLAMF3, CD27ligand/TNFSF7, CD27/TNFRSF7, CD28, CD29, CD2F-10/SLAMF9, CD30ligand/TNFSF8, CD30/TNFRSF8, CD300a/LMIR1, CD4, CD40ligand/TNFSF5, CD40/TNFRSF5, CD48/SLAMF2, CD49a, CD49D, CD49f, CD5, CD53, CD58/LFA-3, CD69, CD7, CD8 α, CD8 β, CD82/Kai-1, CD84/SLAMF5, CD90/Thy1, CD96, CDS, CEACAM1, CRACC/SLAMF7, CRTAM, CTLA-4, DAP12, Dectin-1/CLEC7A, DNAM1 (CD226), DPPIV/CD26, DR3/TNFRSF25, EphB6, GADS, Gi24/VISTA/B7-H5, GITR Ligand/TNFSF18, GITR/TNFRSF18, HLA Class I, HLA-DR, HVEM/TNFRSF14, IA4, ICAM-1, ICOS/CD278, Ikaros, IL2R , IL2R , IL7R , Integrin 4/CD49d, Integrin 4 1, Integrin 4 7/LPAM-1, IPO-3, ITGA4, ITGA6, ITGAD, ITGAE, ITGAL, ITGAM, ITGAX, ITGB1, ITGB2, ITGB7, KIRDS2, LAG-3, LAT, LIGHT/TNFSF14, LTBR, Ly108, Ly9(CD229), lymphocyte function associated antigen-1 (LFA-1), Lymphotoxin-α/TNF-β, NKG2C, NKG2D, NKp30, NKp44, NKp46, NKp80 (KLRF1), NTB-A/SLAMF6, OX40 Ligand/TNFSF4, OX40/TNFRSF4, PAG/Cbp, PD-1, PDCD6, PD-L2/B7-DC, PSGL1, RELT/TNFRSF19L, SELPLG (CD162), SLAM (SLAMF1), SLAM/CD150, SLAMF4 (CD244), SLAMF6 (NTB-A), SLAMF7, SLP-76, TACI/TNFRSF13B, TCL1A, TCL1B, TIM-1/KIM-1/HAVCR, TIM-4, TL1A/TNFSF15, TNF RII/TNFRSF1B, TNF-α, TRANCE/RANKL, TSLP, TSLP R, VLA1, and VLA-6. In some embodiments, the intracellular signaling domain comprises multiple co-stimulatory domains, for example at least two, e.g., at least 3, 4, or 5 co-stimulatory domains. Co-stimulatory signaling regions may provide a signal synergistic with the primary effector activation signal and can complete the requirements for activation of a T cell. In some embodiments, the addition of co-stimulatory domains to the CAR can enhance the efficacy and persistence of the immune cells provided herein.

Binding of the CAR to the B cell surface protein can enhance the proliferation of immune cells, as compared to immune cells lacking a CAR. Proliferation of the immune cells can refer to expansion of the immune cell. Proliferation of the immune cells can refer to phenotypic changes of the immune cell. Proliferation of an immune cell comprising a CAR provided herein can be greater than that of a comparable immune cell lacking the CAR which exhibits binding to a B cell surface protein. Proliferation of an immune cell comprising the CAR can be about 5 fold to about 10 fold, about 10 fold to about 20 fold, about 20 fold to about 30 fold, about 30 fold to about 40 fold, about 40 fold to about 50 fold, about 50 fold to about 60 fold, about 60 fold to about 70 fold, about 70 fold to about 80 fold, about 80 fold to about 90 fold, about 90 fold to about 100 fold, about 100 fold to about 200 fold, from about 200 fold to about 300 fold, from about 300 fold to about 400 fold, from about 400 fold to about 500 fold, from about 500 fold to about 600 fold, from about 600 fold to about 700 fold greater than the proliferation of a comparable immune cell lacking the CAR. Proliferation of an immune cell comprising the CAR can be about 5 fold to about 10 fold, about 10 fold to about 20 fold, about 20 fold to about 30 fold, about 30 fold to about 40 fold, about 40 fold to about 50 fold, about 50 fold to about 60 fold, about 60 fold to about 70 fold, about 70 fold to about 80 fold, about 80 fold to about 90 fold, about 90 fold to about 100 fold, about 100 fold to about 200 fold, from about 200 fold to about 300 fold, from about 300 fold to about 400 fold, from about 400 fold to about 500 fold, from about 500 fold to about 600 fold, from about 600 fold to about 700 fold greater than the proliferation of a comparable immune cell lacking the CAR, and wherein the proliferation is ascertained at least about 12, 24, 36, 48, 60, 72, 84, or 96 hours after contacting the B cell to the B cell surface protein. The enhanced proliferation can be ascertained either in vitro or in vivo. In some embodiments, proliferation can comprise quantifying the number of immune cells. Quantifying a number of immune cells can comprise flow cytometry, Trypan Blue exclusion, and/or hemocytometry. Proliferation can also be determined by phenotypic analysis of the immune cells.

It is worth noting that the purpose of the CD19 CAR modification performed by the present invention is to assist in the expansion and activation of immune cells (e.g., T cells) by binding to B cells. This is not identical to the purpose and function of loading a CD19 CAR for T cells in the common CD19 CAR-T technology, with the most important difference being that the modified immune cells of the present invention are not limited to the treatment of tumors associated with CD19-expressing B cells, as the role of the CD19 CAR in the present invention is not the recognition and binding of target cells.

### Preparation Methods

Any suitable delivery method can be used for introducing compositions and molecules (e.g., polypeptides and/or nucleic acid encoding polypeptides of the disclosure) into a host cell, such as an immune cell. The various components can be delivered simultaneously or temporally separated. The choice of method can be dependent on the type of cell being transformed and/or the circumstances under which the transformation is taking place (e.g., in vitro, ex vivo, or in vivo).

A method of delivery can involve introducing into a cell or a population of cells one or more nucleic acids comprising a nucleotide sequence encoding an enhanced receptor of the invention or other modifying molecule such as a chimeric antigen receptor. Suitable nucleic acids comprising nucleotide sequences encoding the nucleotide sequences may comprise expression vectors, wherein expression vectors comprising nucleotide sequences encoding one or more enhanced receptors or other modifying molecules of the invention are recombinant expression vectors.

Non-limiting examples of delivery methods or transformation include, for example, viral or bacteriophage infection, transfection, conjugation, protoplast fusion, lipofection, electroporation, calcium phosphate precipitation, polyethyleneimine (PEI)-mediated transfection, DEAE-dextran mediated transfection, liposome-mediated transfection, particle gun technology, calcium phosphate precipitation, direct micro injection, and nanoparticle-mediated nucleic acid delivery.

In some aspects, the present disclosure provides methods comprising delivering one or more polynucleotides, or one or more vectors as described herein, or one or more transcripts thereof, and/or one or proteins transcribed therefrom, to a host cell. In some aspects, the disclosure further provides cells produced by such methods, and organisms (such as animals, plants, or fungi) comprising or produced from such cells.

Conventional viral and non-viral based gene transfer methods can be used to introduce nucleic acids in mammalian cells or target tissues. Such methods can be used to administer nucleic acids encoding compositions of the disclosure to cells in culture, or in a host organism. Non-viral vector delivery systems can include DNA plasmids, RNA (e.g. a transcript of a vector described herein), naked nucleic acid, and nucleic acid complexed with a delivery vehicle, such as a liposome. Viral vector delivery systems can include DNA and RNA viruses, which can have either episomal or integrated genomes after delivery to the cell.

Methods of non-viral delivery of nucleic acids can include lipofection, nucleofection, microinjection, biolistics, virosomes, liposomes, immunoliposomes, polycation or lipid: nucleic acid conjugates, naked DNA, artificial virions, and agent-enhanced uptake of DNA. Cationic and neutral lipids that are suitable for efficient receptor-recognition lipofection of polynucleotides can be used. Delivery can be to cells (e.g. in vitro or ex vivo administration) or target tissues (e.g. in vivo administration). The preparation of lipid: nucleic acid complexes, including targeted liposomes such as immunolipid complexes, can be used.

RNA or DNA viral based systems can be used to target specific cells in the body and trafficking the viral payload to the nucleus of the cell. Viral vectors can be administered directly (in vivo) or they can be used to treat cells in vitro, and the modified cells can optionally be administered (ex vivo). Viral based systems can include retroviral, lentivirus, adenoviral, adeno-associated and herpes simplex virus vectors for gene transfer. Integration in the host genome can occur with the retrovirus, lentivirus, and adeno-associated virus gene transfer methods, which can result in long term expression of the inserted transgene. High transduction efficiencies can be observed in many different cell types and target tissues.

The tropism of a retrovirus can be altered by incorporating foreign envelope proteins, expanding the potential target population of target cells. Lentiviral vectors are retroviral vectors that can transduce or infect non-dividing cells and produce high viral titers. Selection of a retroviral gene transfer system can depend on the target tissue. Retroviral vectors can comprise cis-acting long terminal repeats with packaging capacity for up to 6-10 kb of foreign sequence. The minimum cis-acting LTRs can be sufficient for replication and packaging of the vectors, which can be used to integrate the therapeutic gene into the target cell to provide permanent transgene expression. Retroviral vectors can include those based upon murine leukemia virus (MuLV), gibbon ape leukemia virus (GaLV), Simian Immuno deficiency virus (SIV), human immuno deficiency virus (HIV), and combinations thereof.

An adenoviral-based systems can be used. Adenoviral-based systems can lead to transient expression of the transgene. Adenoviral based vectors can have high transduction efficiency in cells and may not require cell division. High titer and levels of expression can be obtained with adenoviral based vectors. Adeno-associated virus (AAV) vectors can be used to transduce cells with target nucleic acids, e.g., in the in vitro production of nucleic acids and peptides, and for in vivo and ex vivo gene therapy procedures.

Packaging cells can be used to form virus particles capable of infecting a host cell. Such cells can include 293 cells, (e.g., for packaging adenovirus), and Psi2 cells or PA317 cells (e.g., for packaging retrovirus). Viral vectors can be generated by producing a cell line that packages a nucleic acid vector into a viral particle. The vectors can contain the minimal viral sequences required for packaging and subsequent integration into a host. The vectors can contain other viral sequences being replaced by an expression cassette for the polynucleotide(s) to be expressed. The missing viral functions can be supplied in trans by the packaging cell line. For example, AAV vectors can comprise ITR sequences from the AAV genome which are required for packaging and integration into the host genome. Viral DNA can be packaged in a cell line, which can contain a helper plasmid encoding the other AAV genes, namely *rep* and *cap,* while lacking ITR sequences. The cell line can also be infected with adenovirus as a helper. The helper virus can promote replication of the AAV vector and expression of AAV genes from the helper plasmid. Contamination with adenovirus can be reduced by, e.g., heat treatment to which adenovirus is more sensitive than AAV. Additional methods for the delivery of nucleic acids to cells can be used, for example, as described in US20030087817, incorporated herein by reference.

A host cell can be transiently or non-transiently transfected with one or more vectors described herein. A cell can be transfected as it naturally occurs in a subject. A cell can be taken or derived from a subject and transfected. A cell can be derived from cells taken from a subject, such as a cell line. In some embodiments, a cell transfected with one or more vectors described herein is used to establish a new cell line comprising one or more vector-derived sequences. In some embodiments, a cell transiently transfected with the compositions of the disclosure (such as by transient transfection of one or more vectors, or transfection with RNA) is used to establish a new cell line comprising cells containing the modification but lacking any other exogenous sequence.

Any suitable vector compatible with the host cell can be used with the methods of the disclosure. Non-limiting examples of vectors for eukaryotic host cells include pXT1, pSG5 (Stratagene TM), pSVK3, pBPV, pMSG, and pSVLSV40 (Pharmacia TM).

Contacting the cells with the composition can occur in any culture media and under any culture conditions that promote the survival of the cells. For example, cells may be suspended in any appropriate nutrient medium that is convenient, such as Iscove's modified DMEM or RPMI 1640, supplemented with fetal calf serum or heat inactivated goat serum (about 5-10%), L-glutamine, a thiol, particularly 2-mercaptoethanol, and antibiotics, e.g. penicillin and streptomycin. The culture may contain growth factors to which the cells are responsive. Growth factors, as defined herein, are molecules capable of promoting survival, growth and/or differentiation of cells, either in culture or in the intact tissue, through specific effects on a transmembrane receptor. Growth factors can include polypeptides and non-polypeptide factors.

In numerous embodiments, the chosen delivery system is targeted to specific tissue or cell types. In some cases, tissue- or cell-targeting of the delivery system is achieved by binding the delivery system to tissue- or cell-specific markers, such as cell surface proteins. Viral and non-viral delivery systems can be customized to target tissue or cell-types of interest.

In preparing a population of modified immune cells for therapeutic use, it is preferred that the proportion of cells with a desired modification such as with TIGIT/OX40, is more than 50% thereof, such as to a level of about 60%, about 70%, about 80%, or even about 90% or higher. It is to be understood that the modified immune cells of the present invention and the associated therapeutic methods are a personalized therapeutic strategy based on an individual patient, and that even when prepared in a similar manner, the proportion of effective cells in the final cellular product may vary due to individualized differences.

### Uses

A variety of target cells can be killed using the immune cells modified with the enhanced receptors of the present invention. A target cell to which this method can be applied includes a wide variety of cell types. A target cell can be in vitro. A target cell can be in vivo. A target cell can be ex vivo. A target cell can be an isolated cell. A target cell can be a cell inside of an organism. A target cell can be an organism. A target cell can be a cell in a cell culture. A target cell can be one of a collection of cells. A target cell can be a mammalian cell or derived from a mammalian cell. A target cell can be a rodent cell or derived from a rodent cell. A target cell can be a human cell or derived from a human cell. A target cell can be a prokaryotic cell or derived from a prokaryotic cell. A target cell can be a bacterial cell or can be derived from a bacterial cell. A target cell can be an archaeal cell or derived from an archaeal cell. A target cell can be a eukaryotic cell or derived from a eukaryotic cell. A target cell can be a pluripotent stem cell. A target cell can be a plant cell or derived from a plant cell. A target cell can be an animal cell or derived from an animal cell. A target cell can be an invertebrate cell or derived from an invertebrate cell. A target cell can be a vertebrate cell or derived from a vertebrate cell. A target cell can be a microbe cell or derived from a microbe cell. A target cell can be a fungi cell or derived from a fungi cell. A target cell can be from a specific organ or tissue.

A target cell can be a stem cell or progenitor cell. Target cells can include stem cells (e.g., adult stem cells, embryonic stem cells, induced pluripotent stem (iPS) cells) and progenitor cells (e.g., cardiac progenitor cells, neural progenitor cells, etc.). Target cells can include mammalian stem cells and progenitor cells, including rodent stem cells, rodent progenitor cells, human stem cells, human progenitor cells, etc. Clonal cells can comprise the progeny of a cell. A target cell can comprise a target nucleic acid. A target cell can be in a living organism. A target cell can be a genetically modified cell. A target cell can be a host cell.

A target cell can be a primary cell. For example, cultures of primary cells can be passaged 0 times, 1 time, 2 times, 4 times, 5 times, 10 times, 15 times or more. Cells can be unicellular organisms. Cells can be grown in culture.

A target cell can be a diseased cell. A diseased cell can have altered metabolic, gene expression, and/or morphologic features. A diseased cell can be a cancer cell, a diabetic cell, and a apoptotic cell. A diseased cell can be a cell from a diseased subject. Exemplary diseases can include blood disorders, cancers, metabolic disorders, eye disorders, organ disorders, musculoskeletal disorders, cardiac disease, and the like.

Of particular interest are cancer cells. In some embodiments, the target cell is a cancer cell. In some embodiments, the targeted cancer cell is a solid tumor cell. In some embodiments, the cancer is of a hematopoietic spectrum, such as a lymphoma. In specific embodiments, the lymphoma is a B-cell lymphoma. The B-cell lymphoma may be a Hodgkin's lymphoma or a non-Hodgkin's lymphoma, including, but not limited to, large B-cell lymphomas such as diffuse large B-cell lymphoma (DLBCL), diffuse large B-cell lymphoma (DLBCL) non-specific (NOS), mediastinal large B-cell lymphoma (PMBCL), follicular cell lymphoma, and mantle cell lymphoma. The tumor may be a refractory tumor or a relapsed tumor, e.g., where the tumor patient has received prior therapy directed to the tumor.

In some embodiments, the target cells form a tumor. A tumor treated with bearing the enhanced receptors of the present invention herein can result in stabilized tumor growth (e.g., one or more tumors do not increase more than 1%, 5%, 10%, 15%, or 20% in size, and/or do not metastasize). In some embodiments, a tumor is stabilized for at least about 1, 2, 3, 4, 5, 6, 7, 8, 9, 10, 11, 12, or more weeks. In some embodiments, a tumor is stabilized for at least about 1, 2, 3, 4, 5, 6, 7, 8, 9, 10, 11, 12, or more months. In some embodiments, a tumor is stabilized for at least about 1, 2, 3, 4, 5, 6, 7, 8, 9, 10, or more years. In some embodiments, the size of a tumor or the number of tumor cells is reduced by at least about 5%, 10%, 15%, 20%, 25, 30%, 35%, 40%, 45%, 50%, 55%, 60%, 65%, 70%, 75%, 80%, 85%, 90%, 95% or more. In some embodiments, the tumor is completely eliminated, or reduced below a level of detection. In some embodiments, a subject remains tumor free (e.g. in remission) for at least about 1, 2, 3, 4, 5, 6, 7, 8, 9, 10, 11, 12, or more weeks following treatment. In some embodiments, a subject remains tumor free for at least about 1, 2, 3, 4, 5, 6, 7, 8, 9, 10, 11, 12, or more months following treatment. In some embodiments, a subject remains tumor free for at least about 1, 2, 3, 4, 5, 6, 7, 8, 9, 10, or more years after treatment.

Death of target cells can be determined by any suitable method, including, but not limited to, counting cells before and after treatment, or measuring the level of a marker associated with live or dead cells (e.g. live or dead target cells). Degree of cell death can be determined by any suitable method. In some embodiments, degree of cell death is determined with respect to a starting condition. For example, an individual can have a known starting amount of target cells, such as a starting cell mass of known size or circulating target cells at a known concentration. In such cases, degree of cell death can be expressed as a ratio of surviving cells after treatment to the starting cell population. In some embodiments, degree of cell death can be determined by a suitable cell death assay. A variety of cell death assays are available, and can utilize a variety of detection methodologies. Examples of detection methodologies include, without limitation, the use of cell staining, microscopy, flow cytometry, cell sorting, and combinations of these.

When a tumor is subject to surgical resection following completion of a therapeutic period, the efficacy of treatment in reducing tumor size can be determined by measuring the percentage of resected tissue that is necrotic (i.e., dead). In some embodiments, a treatment is therapeutically effective if the necrosis percentage of the resected tissue is greater than about 20% (e.g., at least about 30%, 40%, 50%, 60%, 70%, 80%, 90%, or 100%). In some embodiments, the necrosis percentage of the resected tissue is 100%, that is, no living tumor tissue is present or detectable.

Exposing a target cell to modified immune cells or populations of immune cells disclosed herein can be conducted either in vitro or in vivo. Exposing a target cell to modified immune cells or populations of immune cells in vitro can be accomplished by co-culturing target cells and immune cells. Target cells and immune cells can be co-cultured, for example, as adherent cells or alternatively in suspension. Target cells and immune cells can be co-cultured in various suitable types of cell culture media, for example with supplements, growth factors, ions, etc. Exposing a target cell to an immune cell or populations of immune cells in vivo can be accomplished, in some cases, by administering the immune cells to a subject, for example a human subject, and allowing the immune cells to localize to the target cell via the circulatory system. In some cases, an immune cell can be delivered to the immediate area where a target cell is localized, for example, by direct injection.

Exposing can be performed for any suitable length of time, for example at least 1 minute, at least 5 minutes, at least 10 minutes, at least 30 minutes, at least 1 hour, at least 2 hours, at least 3 hours, at least 4 hours, at least 5 hours, at least 6 hours, at least 7 hours, at least 8 hours, at least 12 hours, at least 16 hours, at least 20 hours, at least 24 hours, at least 2 days, at least 3 days, at least 4 days, at least 5 days, at least 6 days, at least 1 week, at least 2 weeks, at least 3 weeks, at least 1 month or longer.

In therapeutic applications, cells or populations of cells having the enhanced receptors of the present invention can be administered to a subject already suffering from a disease or condition, in an amount sufficient to cure or at least partially arrest the symptoms of the disease or condition, or to cure, heal, improve, or ameliorate the condition. Amounts effective for this use can vary based on the severity and course of the disease or condition, previous therapy, the subject's health status, weight, and response to the drugs, and the judgment of the treating physician.

When used for therapeutic purposes, the immune cells of the present invention with enhanced receptors may be administered to a subject at a dose of from about 1 x 10⁷ to about 1 x 10¹² cells, preferably from about 1 x 10⁸ to about 1 x 10¹¹ cells, e.g., about 1 x 10⁷, about 1 x 10⁸, about 1 x 10⁹, about 1 x 10¹⁰, about 1 x 10¹¹ , about 1 x 10¹². In some embodiments, the cell dose is the total amount of cells administered.

The dose of therapeutic cells administered can also be determined based on the weight of the subject. Specifically, the immune cells of the present invention with enhanced receptors may be administered at a dose of about 1 x 10⁵ to about 1 x 10¹¹ cells/kg of subject body weight, preferably about 1 x 10⁶ to about 1 x 10⁸ cells/kg of subject body weight, e.g., about 1 x 10⁶ cells/kg of subject body weight, about 3.3 x 10⁶/kg of subject body weight, about 5 x 10⁶/kg of subject body weight, about 6.7 x 10⁶/kg subject body weight, about 1 x 10⁷ cells/kg subject body weight, about 3.3 x 10⁷/kg subject body weight, about 5 x 10⁷/kg subject body weight, about 6.7 x 10⁷/kg subject body weight, about 1 x 10⁸ cells/kg subject body weight.

The immune cells of the present invention having dual enhanced receptors have a lower effective dose, as compared to immune cells with only a single enhanced receptor. In some embodiments, the immune cells with dual enhanced receptors are capable of achieving similar or better therapeutic effects at doses that are at least 50% lower, or even an order of magnitude lower, compared to immune cells with only a single enhanced receptor. The dual enhanced receptors are preferably a combination of the TIGIT/OX40 enhanced receptor of the invention and another different enhanced receptor, such as a PD1/CD28 enhanced receptor or a PD1(108)/CD28 enhanced receptor.

### Examples

For a fuller understanding and application of the present invention, the invention will be described in detail below with reference to embodiments and drawings, the embodiments being intended to exemplify the invention only, and not intended to limit the scope of the invention. The scope of the present invention is specifically limited by the attached items.

### Example 1. Preparation of lentiviral vectors expressing enhanced receptors

In order to determine the effect of the TIGIT/OX40 enhanced receptor of the present invention versus other enhanced receptors, the following different lentiviral vectors were prepared using a fourth generation lentiviral vector system (tetra plasmid system) transfected into 293T cells by PEI (Polysciences, 23966-1) reagent transfection reagent:
1) NY-ESO-1 TCR: an artificial TCR targeting the tumor target NY-ESO-1;
2) CD19 CAR: a CAR targeting CD19;
3) TIGIT/OX40: an enhanced receptor, wherein the ECD is derived from TIGIT, ICD for OX40, and TM for OX40;
4) TIGIT/OX40-TCR: the TIGIT/OX40 gene in 3) above and the artificial TCR gene targeting the tumor target NY-ESO-1 in 1) above were constructed in the same vector, and the two gene sequences is linked by a T2A sequence, the TIGIT/OX40 enhanced receptor protein and the TCR protein can be expressed individually on the cell membrane by shearing the T2A sequence by shear enzymes, respectively, creating a non-fusion expression of the two proteins;
5) TIGIT/OX40-CAR: non-fusion expression of the TIGIT/OX40 in 3) above and the CAR targeting CD19 in 2) above;
6) TIGIT/CD28-TCR: non-fusion expression of the enhanced receptor TIGIT/CD28 and the artificial TCR targeting the tumor target NY-ESO-1, wherein the ECD of the enhanced receptor TIGIT/CD28 was derived from TIGIT and the ICD and TM were derived from CD28;
7) PD1/CD28-TCR: non-fusion expression of the enhanced receptor PD1/CD28 and the artificial TCR targeting the tumor target NY-ESO-1, wherein the ECD of the enhanced receptor PD1 was derived from wild-type PD1, and the ICD and TM were derived from CD28.

The preparation of the corresponding lentiviral vector was illustrated by the example of TIGIT/OX40-TCR. The purpose of loading the TCR for the cells was to make them recognize tumor cells with the corresponding antigen. This is because in animal (mouse) experiments, the tumor in the mouse was a xenograft from a human being, and TCR modification enabled the immune cells for therapeutic use to recognize the xenograft, thereby mimicking the immune cells that have tumor-recognizing properties prior to being modificated in practical applications. It should be appreciated that, in practice, tumor cell recognition properties can be conferred not only by loading a TCR (e.g., preparing a neoantigen-recognizing TCR based on an individual's mutational profile), but also by isolating tumor-infiltrating lymphocytes (TILs), and by isolating PD1⁺ T-cells from the peripheral blood, in order to obtain cells having tumor recognition properties. In the latter two cases, loading of tumor antigen or neoantigen-recognizing TCRs is not necessary, and thus this example also described protocols for preparing TIGIT/OX40 enhanced receptors alone.

First, a primary vector expressing the TIGIT/OX40-TCR construct was constructed. The TIGIT/OX40-TCR construct was constructed by spacing the TIGIT/OX40 enhanced receptor gene sequence and the TCR gene sequence in the same vector by the T2A sequence, and the TIGIT/OX40 consisted of the TIGIT extracellular domain (TIGIT ECD), the OX40 transmembrane domain (OX40 TM) and the OX40 intracellular domain (OX40 ICD), the sequences of which are shown in Table 1 below.

**Table 1. Sequence composition of TIGIT/OX40-TCR constructs**

| | Coding Sequence | Amino acid sequence |
|---|---|---|
| TIGIT ECD | SEQ ID NO: 1 | SEQ ID NO: 9 |
| OX40 ICD | SEQ ID NO: 2 | SEQ ID NO: 10 |
| OX40 TM | SEQ ID NO: 3 | SEQ ID NO: 11 |
| TCR | SEQ ID NO: 18 | SEQ ID NO: 17 |
| T2A | SEQ ID NO: 22 | SEQ ID NO: 21 |

The primary vector TIGIT/OX40-TCR plasmid, and packaging vector pMDL-gag plasmid, Rev plasmid, and envelope vector pMD2.G plasmid, were mixed in the ratio of 1:2:2:1, after which 293T (ATCC) cells were co-transfected using PEI reagent. After the transfected cells were cultured at 37°C for 48 h, the supernatant was collected and ultracentrifugation was performed to concentrate the lentivirus. The lentiviral pellet obtained by centrifugation was resuspended with 1 mL of 1640 medium and dispensed into 10 1.5 ml EP tubes of 100 µl each, and stored at -80°C.

The titer of TIGIT/OX40-TCR enhanced receptor lentiviral vector was tested. 100 µl of the viral stock solution was taken and diluted 3, 9, 27, 81, 243, and 729 times using a 3-fold multiplicative dilution, after which 50 µl of each viral sample from each different dilution was taken to infect the 293T cells for 48 hours to 72 h.

The culture supernatant was removed, the cells were washed with PBS and trypsin was added. The 293T cells were collected and stained for TIGIT with an anti-TIGIT antibody (Biolegend, 372714), and the proportion of TIGIT⁺ cells in the virus-infected cells at each of the different dilutions was analyzed by flow cytometry and the titer was calculated according to the following formula: Titer (TU/ml) = number of starting 293T cells (about 40,000 to 45,000) × percentage of TIGIT+ cells × Fold of dilutions × 20 (the first set of viral dilutions in which the percentage of TIGIT⁺ cells was <20% was taken for calculation).

Using a similar approach, the 7 constructs listed above were constructed with the amino acid and nucleotide sequences shown below. Lentiviral vectors with these constructs were used to transfect 293T cells.

**Table 2: Sequence composition of other elements**

| | Coding Sequence | Amino acid sequence |
|---|---|---|
| NY-ESO-1 TCR | SEQ ID NO: 18 | SEQ ID NO: 17 |
| CD19 CAR | SEQ ID NO: 20 | SEQ ID NO: 19 |
| CD28 ICD | SEQ ID NO: 5 | SEQ ID NO: 13 |
| CD28 TM | SEQ ID NO: 6 | SEQ ID NO: 14 |
| PD1 ECD | SEQ ID NO: 8 | SEQ ID NO: 16 |

Following the method as described above, the proportion of antibody-labeled positive cells was detected by flow cytometry through different antibody labels, and the titer of each lentivirus was calculated, and if the titer was >3 × 10⁷ it was ready to be used in the next step.

### Example 2. Preparation of tumor-recognizing immune cells loaded with enhanced receptors

In this Example, T cells are used as the immune cells bearing enhanced receptors, and T cells were modified to provide specific recognition properties for specific tumor cells, thereby achieving specific binding of immune cells to tumor cells in vitro experiments.
In order to compare the effect of different enhanced receptors, a total of four different tumor-recognizing T cells were prepared, each bearing a NY-ESO-1-TCR that specifically recognizing NY-ESO-1, wherein the ii) to iv) bearing enhanced receptor modifications. The details are as follows:
i) NY-ESO-1-TCR-T: T cells modified to express NY-ESO-1-TCR, which can target tumor cells expressing NY-ESO-1 by an artificial TCR, also referred to as TCR-T;
ii) TIGIT/OX40-TCR-T: T cells modified to express the enhanced receptor TIGIT/OX40 and NY-ESO-1-TCR, which can target to tumor cells expressing NY-ESO-1 by an artificial TCR;
iii) TIGIT/CD28-TCR-T: T cells modified to express the enhanced receptor TIGIT/CD28 and NY-ESO-1-TCR, which can target to tumor cells expressing NY-ESO-1 by an artificial TCR;
iv) PD1/CD28-TCR-T: T cells modified to express the enhanced receptor PD1/CD28 and NY-ESO-1-TCR, which can target to tumor cells expressing NY-ESO-1 by an artificial TCR.

As described above, the TCR conferred tumor recognition properties to the immune cells, and various enhanced receptors were used to increase the potency of the immune cells.

Peripheral blood single nucleated cells were apheresised from tumor patients, and PD1⁺ cells were isolated from the apheresised cells and divided equally into 5 groups. CD3/CD28 dynabeads were added to each group separately for overnight stimulation, and the next day lentiviral vectors comprising 1) NY-ESO-1-TCR, 4) TIGIT/OX40-TCR, 6) TIGIT/CD28-TCR, and 7) PD1/CD28-TCR from Example 1 were added to each of the 4 groups of cells according to MOI (number of lentiviruses/number of cells)=2, respectively. The above transfected cells (TCR-T, TIGIT/OX40-TCR-T, TIGIT/CD28-TCR-T, and PD1/CD28-TCR-T cells) were cultured and expanded, and T cells without the addition of any viral transfection were used as a control (Mock-T).

### Example 3. Effect of TIGIT/OX40 enhanced receptor on activation of T cells in vitro

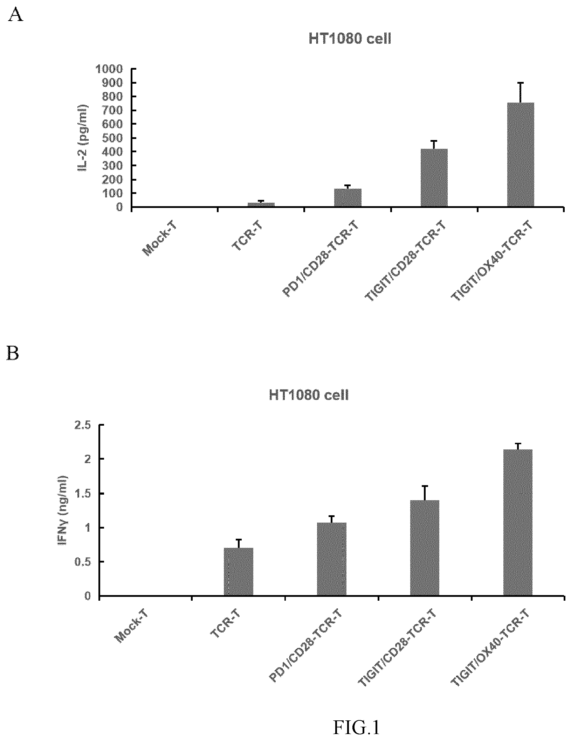

To verify the function of the TIGIT/OX40 enhanced receptor, the tumor-associated antigen NY-ESO-1 was used as a target, and the human fibrosarcoma cell line HT1080 (a gift from Beijing JOINN Laboratories (China) Co., Ltd.), which naturally expresses NY-ESO-1, was used as a target cell, and the HLA-A*02:01 gene was expressed in HT1080 cells. All four cells constructed in Example 2 express TCR-T targeting NY-ESO-1, and these cells were used as effector T cells. The activation level of the T cells with different modifications was thereby determined by co-culturing the tumor cells HT1080 with the T cells and detecting the amount of IFN-γ and IL-2 secreted by the T cells, and the results are shown in FIGS. 1A-B.

As shown in FIGS. 1A-B, IL-2 and IFN-γ secreted by TIGIT/OX40-TCR-T cells were significantly higher than those secreted by PD1/CD28-TCR-T, TIGIT/CD28-TCR-T, and TCR-T cells. The results of the in vitro experiments showed that immune activation of TCR-T cells expressing the enhanced receptors was stronger in the HT1080 tumor cell model, and that the two enhanced receptors with TIGIT as the extracellular domain were superior to the enhanced receptors with PD1 as the extracellular domain, with the highest level of activation by TIGIT/OX40.

### Example 4. Effect of TIGIT/OX40 enhanced receptor on activation of T cells in vivo

In order to further verify the role of TIGIT/OX40 enhanced receptor, a J82-NYESO1 (J82-NY) tumor cell line was first constructed by load expressing NY-ESO-1 tumor antigen in human bladder metastatic carcinoma cells J82 (purchased from the Chinese Academy of Medical Sciences, item number 1101HUM-PUMC000346). Specifically, lentivirus carrying NY-ESO-1 was added to J82 cells according to MOI=5, and G418 was added 72 hours later to screen positive cells, and the expression of NY-ESO-1 was detected by flow cytometry about 2 weeks later. Flow cytometry results showed that up to 95% or more of J82 cells expressed NY-ESO-1, indicating that the cell construction was successful. Subsequently, a xenograft mouse model of J82-NY tumor cells was constructed.

1x10⁶ tumor cells J82-NY were subcutaneously inoculated into NSG mice (purchased from Beijing IDMO co., Ltd.). When the tumor volume reached 80~120 mm³, 30 mice were selected and randomly divided into the following 6 groups (5 mice in each group), and given one of the following 6 cell treatments:
a. Group of freezing medium(Freezing medium group);
b. Group of untransduced T cells (Mock-T group);
c. Group of TCR cells transduced with NY-ESO-1 only (TCR-T group);
d. Group of TCR cells transduced with PD1/CD28 enhanced receptor and NY-ESO-1 (PD1/CD28-TCR-T group);
e. Group of TCR cells transduced with TIGIT/CD28 enhanced receptor and NY-ESO-1 (TIGIT/CD28-TCR-T group);
f. Group of TCR cells transduced with TIGIT/OX40 enhanced receptor and NY-ESO-1 (TIGIT/OX40-TCR-T group).

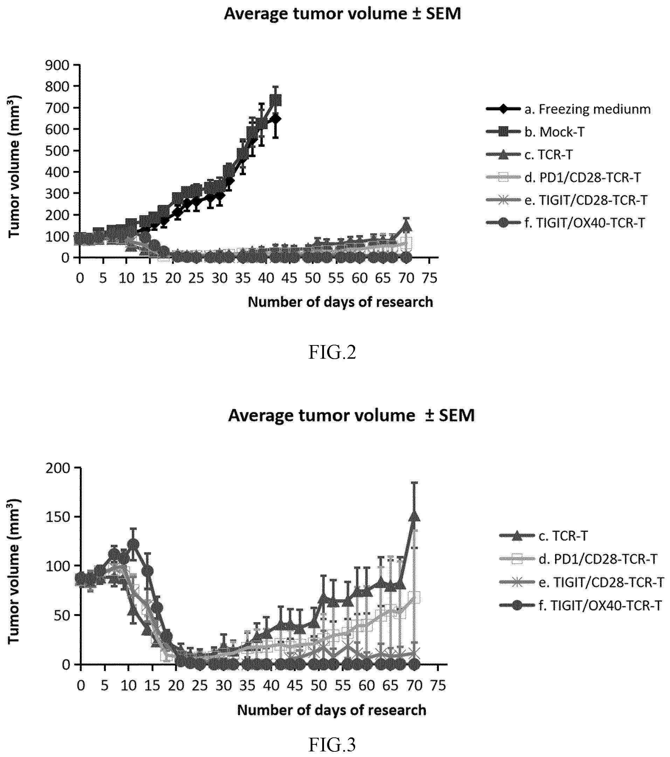

Each group was administered as a single tail vein infusion of 200 µl totaling 8 x 10⁶ cells (effective cell number of 6 × 10⁶), and the control group was given 0.2 ml of cell freezing medium accordingly. After administration, the measurements of the tumor size and the observations of the state of the mice were carried out every 2-3 days. Tumor volume was calculated according to "Tumor volume=1/2×long diameter×short diameter×short diameter", and the average tumor volume of 5 mice in each group was calculated. The observation was continued for 70 days, and the results were shown in FIG. 2 and FIG. 3.

As shown in FIG. 2 and FIG. 3, the tumor volumes of mice in the freezing medium and Mock-T groups continued to grow. The mice in both groups were euthanized due to excessive tumor volume on the 44th day of observation. The tumor volumes of mice in groups c, d, e, and f, which were administered tumor-recognizing TCR-T cells, began to shrink after the 10th day and showed the strongest efficacy around day 25. However, as the observation time was extended, the tumors of the mice in the TCR-T group began to recur, and started to grow progressively larger after day 35, and tumor progression was observed in 4 of the 5 mice in the TCR-T group on day 70, when the tumor volume exceeded that at the start of treatment, and the other mouse was found to be dead on day 70. The average tumor volume of mice in PD1/CD28-TCR-T group also continued to increase after day 46. The efficacy was better maintained in the TIGIT/CD28-TCR-T group and the TIGIT/OX40-TCR-T group. The results of the enlarged FIG. 3 show that the tumors of the mice in the TIGIT/OX40 group were maintained the state of completely cleared and did not recur until the end of the observation, while some mice in the TIGIT/CD28 group showed tumor recurrence.

The above results indicate that TIGIT/OX40 not only enhances the tumor suppressor effect of TCR-T, but also has a stronger function in the maintenance of T cell function and efficacy than the other two enhanced receptors.

### Example 5. Experiment 1 on the association of enhanced receptors TIGIT/OX40 and PD1/CD28

This Example involves the simultaneous modification of T cells with two enhanced receptors. Said two enhanced receptors are (1) the enhanced receptor PD1(108)/CD28 constructed based on PD1 mutant 108#, and (2) TIGIT/OX40 as described in Example 1.

### Preparation of lentiviral vectors expressing the PD1(108)/CD28-TIGIT/OX40 dual enhanced receptor

Compared to wild-type PD1, PD1 mutant 108# was able to bind human PD-L1 with high affinity, but not human PD-L1 including Sintilimab, Nivolumab, Camrelizumab, Pemrolizumab, and Toripalimab, among many other anti-PD1 antibodies.

In order to enable T cells to express both two enhanced receptors, a nucleotide sequence encoding PD1(108)/CD28 was ligated with a nucleotide sequence encoding TIGIT/OX40 using a nucleotide sequence encoding T2A (SEQ ID NO: 22), which was introduced into a lentiviral vector as the dual enhanced receptor construct PD1(108)/CD28-TIGIT/OX40.

In this way, after transfection with the lentiviral system, with the aid of the self-cleaving peptide T2A, the two enhanced receptors will be expressed in the transfected T cells, and the two enhanced receptors will function separately to co-regulate the T. The amino acid sequence and the coding nucleotide sequence of PD1(108) were shown in SEQ ID NO: 12 and SEQ ID NO: 4, respectively.

Lentiviral vectors were constructed in the same manner as Example 1 and used to transfect 293T cells, and then titers were measured. Specifically, lentiviral vectors comprising the following gene sequences were constructed:
1) PD1(108)/CD28-TIGIT/OX40-TCR: non-fusion expression of the dual enhanced receptor construct PD1(108)/CD28-TIGIT/OX40 and the artificial TCR targeting the tumor target NY-ESO-1 as described in Example 1.

The titer of each lentivirus was calculated and if the titer was >3×10⁷, it was ready for next use.

### Effect of PD1(108)/CD28-TIGIT/OX40 dual enhanced receptor expression in activating T cells in vitro

To verify the function of the PD1(108)/CD28-TIGIT/OX40 dual enhanced receptor, J82-NY cells overexpressing NY-ESO-1 were used as target cells and co-cultured with T cells bearing a TCR specifically recognizing NY-ESO-1, and the amount of IFN-γ and IL-2 secreted by T cells with different modifications was measured, thereby detecting the effect of the enhanced receptor modifications on the degree of T cells activation.

In order to compare the effect of different enhanced receptors, in a similar manner as in Example 2, a total of four different tumor-recognizing T cells were prepared, each bearing the NY-ESO-1-TCR that specifically recognizing NY-ESO-1, and the ii) to iv) further bearing different enhanced receptor modifications. The details are as follows:
i) NY-ESO-1-TCR-T: T cells modified to express NY-ESO-1-TCR, which can target tumor cells expressing NY-ESO-1 by an artificial TCR, also referred to as TCR-T;
ii) PD1/CD28-TCR-T: T cells modified to express the enhanced receptors PD1/CD28 and NY-ESO-1-TCR, which can target to tumor cells expressing NY-ESO-1 by an artificial TCR;
iii) antiPDL1/CD28-TCR-T: T cells modified to express the enhanced receptor antiPDL1/CD28 and NY-ESO-1-TCR, which can target to tumor cells expressing NY-ESO-1 by an artificial TCR, wherein antiPDL1 represents that this enhanced receptor has an anti-PD-L1 single-chain antibody (PD-L1 scFv) as ECD, and the amino acid sequence and nucleotide sequence of which are shown in SEQ ID NO: 15 and SEQ ID NO: 7, respectively;
iv) PD1(108)/CD28-TIGIT/OX40-TCR-T: T-cells modified to express two enhanced receptors PD1(108)/CD28 and TIGIT/OX40-TCR-T, which can target to tumor cells expressing NY-ESO-1 by an artificial TCR.

The cells of species i) and ii) above are prepared as described in Examples 1 and 2. The preparation of cells of species iii) was similar to that of species ii) in that both enhanced receptors bind PD-L1, but ii) used the receptor PD-1 for PD-L1 ligand as the ECD, whereas iii) used scFv for anti-PD-L1 antibody as the ECD. The preparation of cells of species iv) is accomplished using a lentiviral vector as described in this Example, following the same transfection process as in Example 2.

The density of J82-NY cells was adjusted to 1×10⁶ cells/mL, and 100 µl of them were put into a U-shaped bottom 96-well plate. According to the ratio of effector cells: target cells=1:1 to J82-NY cells, 100 µl of each of the above four T cells was taken and added into the U-shaped bottom 96-well plate with J82-NY cells. After 24 hours of co-culture at 37°C, the plates were put into a centrifuge and centrifuged at 400g for 5 minutes. Take 150 µl of culture supernatant and transfer it to a new 96-well plate, taking care not to aspirate the cells at the bottom. After aspirating the supernatant, the plate was sealed around with sealing film and stored at 4°C. ELISA was performed the next day.

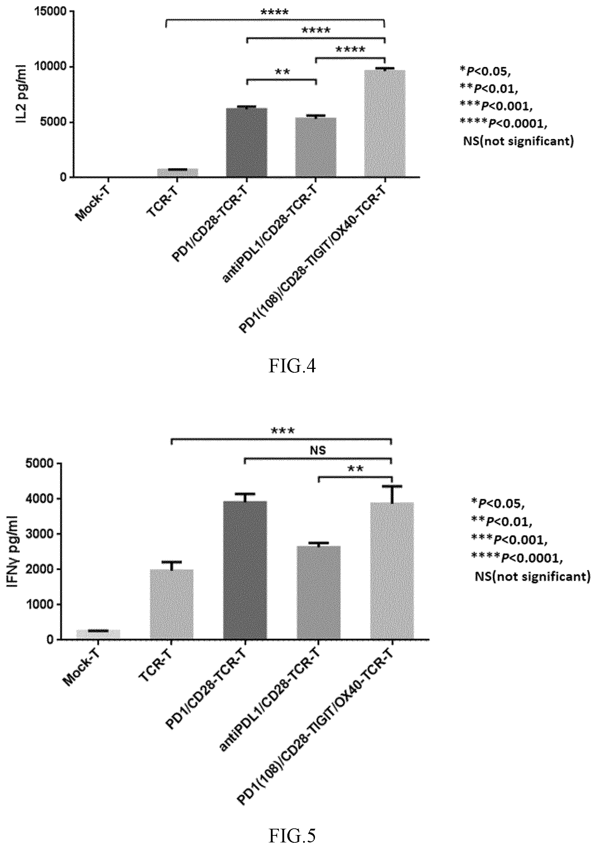

ELISA was performed using a human IL-2 detection kit (ELISA method)(Dakewe Biotech Co., Ltd., Product code 1110203) according to the following procedure: The microtiter plate was pre-coated with anti-human IL-2 capture antibody. To the microtiter plate, standards or samples to be tested and detection antibody (biotin-labeled anti-human IL-2 antibody) were added at the same time, and then horseradish peroxidase-labeled streptavidin (SA-HRP), which specifically binds biotin in the immune complex, was added. The enzyme substrate tetramethylbenzidine (TMB) was added, and blue color appeared, the shade of the color correlated with the concentration of IL-2 in the standards or samples. After 5-10 minutes of color development, the reaction was terminated by adding a termination solution, and the light absorption value (OD) at 450 nm was read by a multi-mode microplate reader. The concentration of human IL-2 was proportional to the OD value within a certain range, and the standard curve was obtained by ELISA using IL-2 standard. The IL-2 concentration in each co-culture was calculated using this standard curve. Results of this experiment are shown in FIG. 4.

ELISA was performed using a human IFNγ detection kit (Dakewe Biotech Co., Ltd., Product code 1110003) according to the following procedure: To the microtiter plate, standards or samples to be tested and detection antibody (biotin-labeled anti-human IFNγ antibody) were added at the same time, and then horseradish peroxidase-labeled streptavidin (SA-HRP), which specifically binds biotin in the immune complex, was added. The enzyme substrate tetramethylbenzidine (TMB) was added, and blue color appeared, the shade of the color correlated with the concentration of IFNγ in the standards or samples. After 5-10 minutes of color development, the reaction was terminated by adding a termination solution, and the light absorption value (OD) at 450 nm was read by a multi-mode microplate reader. The concentration of human IFNγ was proportional to the OD value within a certain range, and the standard curve was obtained by ELISA using IFNγ standard. The IFNγ concentration in each co-culture was calculated using this standard curve, and the results were shown in FIG. 5.

As shown in FIG. 4, the IL-2 secreted by PD1(108)/CD28-TIGIT/OX40-TCR-T cells was significantly higher than IL-2 secreted by PD1/CD28-TCR-T, antiPDL1/CD28-TCR-T and TCR-T cells. As the results shown in FIG.5, the IFN-y secreted by PD1(108)/CD28-TIGIT/OX40-TCR-T cells was significantly higher than IFN-γ secreted by antiPDL1/CD28-TCR-T and TCR-T cells, while there was no difference in the amount of IFN-γ secreted from PD1/CD28-TCR-T cells. The results of the in vitro experiments indicated that TCR-T cells with dual enhanced receptor modifications achieved significantly higher levels of activation.

### Animal experiments with NY-ESO-1 TCR-T cells expressing PD1(108)/CD28-TIGIT/OX40 enhanced receptors

To verify the role of the PD1(108)/CD28-TIGIT/OX40 dual enhanced receptor, in vivo mouse experiments were performed using a method similar to that in Example 4.

1×10⁶ tumor cells, J82-NY, were subcutaneously inoculated into NSG mice. When the tumor volume reached 80-120 mm³, 25 mice were selected and randomly divided into the following 5 groups (5 mice per group) and given one of the following 5 cell treatments, respectively:
a. Group of untransduced T cells (Mock-T group);
b. Group of TCR cells transduced with NY-ESO-1 only (TCR-T group);
c. Group of TCR cells transduced with PD1/CD28 enhanced receptor and NY-ESO-1 (PD1/CD28-TCR-T group);
d. Group of TCR cells transduced with antiPDL1/CD28 enhanced receptor and NY-ESO-1 (antiPDL1/CD28-TCR-T group);
e. Group of TCR cells transduced with PD1(108)/CD28-TIGIT/OX40 enhanced receptor and NY-ESO-1 (PD1(108)/CD28-TIGIT/OX40-TCR-T group).

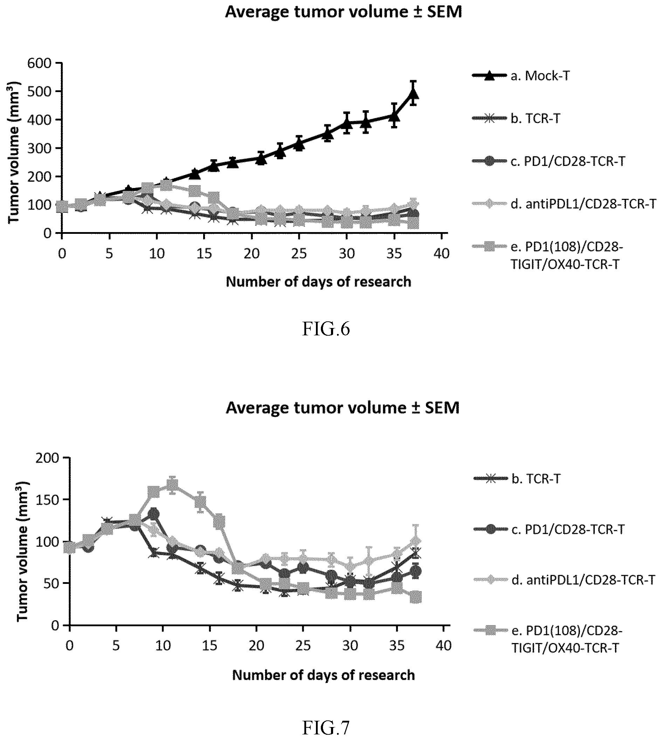

Each group was administered by a single tail vein infusion of 6 × 10⁶ TCR-positive active cells, except for the Mock-T group, where the amount of cells infused into the tail vein of each mouse in the Mock-T group was the same as the total amount of cells being given to each mouse in the remaining groups. After administration, the measurements of the tumor size and the observations of the state of the mice were carried out every 2-3 days. Tumor volume was calculated according to "Tumor volume=1/2×long diameter×short diameter×short diameter". The observation was continued for 37 days, and the results were shown in FIG. 6 and FIG. 7.

The results were shown in FIG. 6 and FIG. 7. The tumor volume of mice in the Mock-T group continued to grow, and the tumor volume of mice in groups b, c, d, and e first increased and then decreased. Tumors in mice in the TCR-T group shrank fastest in the early phase, but began to grow progressively larger with tumor progression after day 28.

The average tumor volume of mice in the PD1/CD28-TCR-T group also continued to increase after day 35. The best efficacy was observed in the PD1(108)/CD28-TIGIT/OX40-TCR-T group. These results suggest that T cells bearing dual enhanced receptors and capable of specifically recognizing tumors provide better tumor suppression in vivo, compared to T cells bearing only enhanced receptors that specifically bind PD-L1.

### Example 6. Experiment 2 on the association of enhanced receptors TIGIT/OX40 and PD1/CD28

Animal experiments were again performed according to methods similar to Example 6 to validate the effect of the coupling of TIGIT/OX40 and PD1(108)/CD28.

### Preparation of lentiviral vectors expressing the PD1(108)/CD28-TIGIT/OX40 dual enhanced receptor

As with the construct of Example 6, the PD1(108)/CD28-TIGIT/OX40 dual enhanced receptor was constructed by joining the coding sequence of PD1(108)/CD28, with the coding sequence of TIGIT/OX40, using the coding sequence of T2A. A safer fourth generation lentiviral vector system was used for transduction. The primary vector PD1(108)/CD28-TIGIT/OX40, packaging vector pMDL-gag, Rev, and envelop vector pMD2.G were used, and co-transfected into HEK293T cells with calcium phosphate or liposome-PE. The supernatant was collected after 48 h, and centrifuged to concentrate the lentivirus.

For titer assay of PD1(108)/CD28-TIGIT/OX40 enhanced receptor lentiviral vectors, dilutions were made using a 3-fold multiplicity and 50 µl were taken to infect 293T cells for 48 h to 72 h. 293T cells were collected for PD1(108) staining. The proportion of PD1(108)-positive cells was analyzed by flow cytometry and titer calculation was performed according to the formula: Titer (TU/ml) = number of starting 293T cells (about 40,000 to 45,000) × percentage of PD1(108)+ cells × Fold of dilutions × 20 (the first set of viral dilutions in which the percentage of PD1(108)⁺ cells was <20% was taken for calculation).

Since the PD1(108)/CD28 enhanced receptor and the TIGIT/OX40 enhanced receptor are on the same vector, the titer of the PD1(108)/CD28 enhanced receptor was used to represent the titer of the TIGIT/OX40 enhanced receptor.

Lentiviral vectors comprising the following gene sequences were constructed in a similar manner:
1) non-fusion expression of the PD1(108)/CD28-TIGIT/OX40 and the artificial TCR targeting the tumor target NY-ESO-1, referred to as PD1(108)/CD28-TIGIT/OX40-TCR;
2) the PD1(108)/CD28 and the artificial TCR targeting the tumor target NY-ESO-1, referred to as PD1(108)/CD28-TCR;
3) the TIGIT/OX40 and the artificial TCR targeting the tumor target NY-ESO-1, referred to as TIGIT/OX40-TCR;
4) the artificial TCR targeting the tumor target NY-ESO-1, referred to as TCR.

The titer of each lentivirus was calculated and if the titer was >3×10⁷, it was ready for next use.

### Animal experiments with NY-ESO-1 TCR-T cells expressing PD1(108)/CD28-TIGIT/OX40 dual enhanced receptors

To verify the role of the PD1(108)/CD28-TIGIT/OX40 dual enhanced receptor, the tumor-associated antigen NY-ESO-1 was used as a target, J82 tumor cells expressing NY-ESO-1 were used as target cells (J82-NY-ESO1, or J82-NY), and TCR-T cells targeting NY-ESO-1 were used as effector T cells , allowing TCR-T cells targeting NY-ESO-1 to simultaneously express PD1(108)/CD28-TIGIT/OX40 enhanced receptor modifications, to prepare enhanced TCR-T cells. 1×10⁶ tumor cells, J82-NY, were subcutaneously inoculated into NSG mice. When the tumor volume reached 80-120 mm³, 30 mice were selected and randomly divided into 6 groups and given the following cell treatments, respectively: a. Group of freezing medium (Freezing medium); b. Group of untransduced T cells (Mock-T group); c. Group of TCR cells transduced with NY-ESO-1 only(TCR-T group); d. Group of TCR cells transduced with PD1(108)/CD28 enhanced receptor and NY-ESO-1 (PD1(108)/CD28-TCR-T group); e. Group of TCR cells transduced with TIGIT/OX40 enhanced receptor and NY-ESO-1 (TIGIT/OX40-TCR-T group); f. Group of TCR cells transduced with PD1(108)/CD28-TIGIT/OX40 enhanced receptor and NY-ESO-1 (PD1(108)/CD28-TIGIT/OX40-TCR-T group); The sequences of the containing elements in each group are the same as above. All were administered at a total cell volume of 1.5×10⁷ cells/each (3×10⁶ TCR-positive cells/each ), and were administered once by tail vein infusion. After administration, the measurements of the tumor size and the observations of the state of the mice were carried out every 2-3 days. Tumor volume was calculated according to "Tumor volume=1/2×long diameter×short diameter×short diameter", and was observed for 31 days.

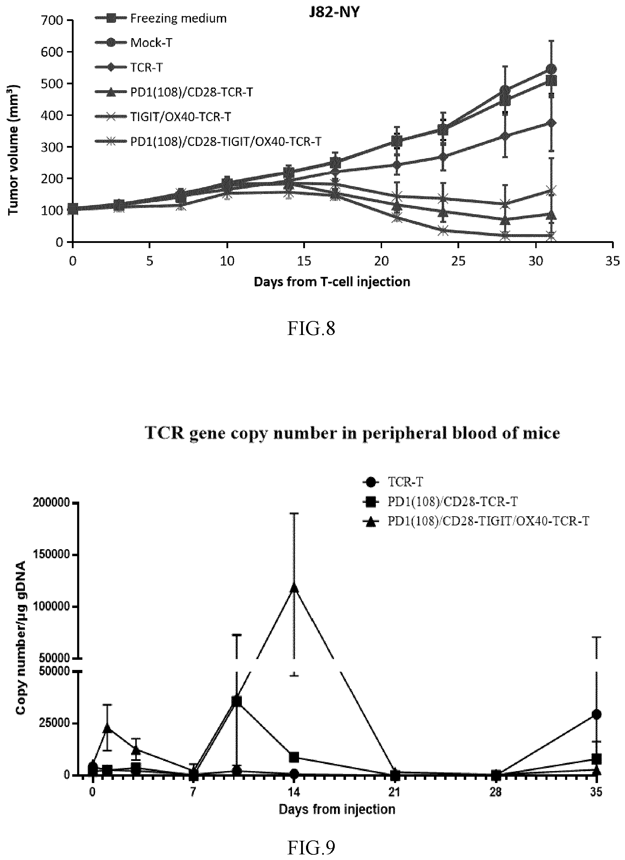

The results are shown in FIG. 8 and Table 3 (wherein the tumor volume is the average value of each group), the tumor volume of the mice in the Freezing medium group and the Mock-T group continued to grow, and the tumor growth rate of the mice in the TCR-T group was slower than that of the Freezing medium group and the Mock-T group, and the growth of the tumors was inhibited to a certain extent. Tumor growth of mice in all 3 groups expressing both enhanced receptor and TCR showed a decrease in tumor volume, suggesting that expression of enhanced receptor promoted the tumor suppressor function of TCR-T cells. Surprisingly, the PD1(108)/CD28-TIGIT/OX40-TCR-T group expressing dual enhanced receptors demonstrated significant tumor suppressive effects with the longest duration of efficacy maintenance. The combination of the two enhanced receptors demonstrated a synergistic effect in tumor suppression compared to cells containing only one of the enhanced receptor modifications.

**Table 3. Tumor volume of different treatment groups at different time points after injection (mm³)**

| Group | D0 | D3 | D7 | D10 | D14 | D17 | D21 | D24 | D28 | D31 |
|---|---|---|---|---|---|---|---|---|---|---|
| Freezing medium | 104.80 | 115.39 | 140.53 | 182.19 | 218.55 | 250.12 | 317.98 | 353.58 | 446.59 | 508.96 |
| Mock-T | 104.60 | 116.80 | 148.34 | 185.80 | 219.99 | 252.53 | 317.43 | 356.70 | 477.84 | 544.86 |
| TCR-T | 104.80 | 119.13 | 144.47 | 164.60 | 192.14 | 221.20 | 243.35 | 268.44 | 333.85 | 375.49 |
| PD1(108)/CD28 -TCR-T | 102.80 | 117.68 | 149.92 | 177.87 | 182.81 | 153.38 | 116.55 | 95.55 | 70.22 | 87.70 |
| TIGIT/OX40-TCR-T | 102.60 | 114.68 | 152.60 | 181.67 | 184.97 | 181.65 | 143.26 | 136.31 | 118.99 | 161.43 |
| PD1(108)/CD28 -TIGIT/OX40-TCR-T | 101.40 | 109.45 | 114.87 | 152.86 | 156.23 | 145.31 | 76.39 | 35.68 | 19.72 | 18.09 |

### In vivo pharmacokinetic study of NY-ESO-1 TCR-T cells expressing PD1(108)/CD28-TIGIT/OX40 enhanced receptor

To verify the role of the PD1(108)/CD28-TIGIT/OX40 enhanced receptor, the situation of the in vivo metabolism of TCR-T cells expressing PD1(108)/CD28-TIGIT/OX40 enhanced receptor was evaluated using the J82-NY tumor model.

1×10⁶ tumor cells, J82-NY, were subcutaneously inoculated into NSG mice. When the tumor volume reached 80-120 mm³, 54 mice were selected and randomly divided into 3 groups and given the following cell treatments, respectively: a. Group of TCR cells transduced with NY-ESO-1 only (TCR-T group); b. Group of TCR cells transduced with PD1(108)/CD28 enhanced receptor and NY-ESO-1 (PD1(108)/CD28-TCR-T group); c. Group of TCR cells transduced with PD1(108)/CD28-TIGIT/OX40 enhanced receptor and NY-ESO-1 (PD1(108)/CD28-TIGIT/OX40-TCR-T group. All were administered at a dose of 5×10⁶ TCR-positive cells/each, and were administered once by tail vein infusion. After being infused back with T cells, two mice were taken from each group at D0 (2 h after infusion), D1, D3, D7, D10, D14, D21, D28, and D35. After the animals were anesthetized with isoflurane, euthanasia was performed, and whole blood (EDTA anticoagulation) and tumor tissues were collected, and the samples were stored at -80°C. The measurements of the tumor size and the observations of the state of the mice were carried out every 2-3 days. Tumor volume was calculated according to "Tumor volume=1/2×long diameter×short diameter×short diameter", and was observed for 35 days. The situation of the metabolism of the administered therapeutic T cells in mice was evaluated by detecting the content of TCR genes in peripheral blood and tumor tissues of different mice by qPCR.

The results, as shown in FIG. 9, TCR-T cells did not show a large proliferation in the peripheral blood of mice for 28 days after reflux, and the expansion peak appeared at D35. In contrast, PD1(108)/CD28-TCR-T cells and PD1(108)/CD28-TIGIT/OX40-TCR-T cells showed expansion peaks at D10 and D14, respectively, and the extents of expansion of PD1(108)/CD28-TIGIT/OX40-TCR-T cells were stronger in peripheral blood than PD1(108)/CD28- TCR-T cells. This result suggests that the enhanced receptor PD1(108)/CD28-TIGIT/OX40 has a stronger promotional effect on the expansion and survival of TCR-T cells in the peripheral blood of mice.

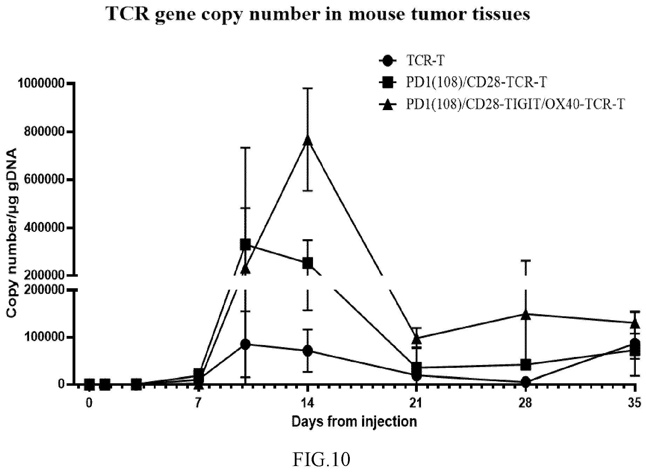

The results, as shown in FIG. 10, after T-cell reinfusion, TCR genes could be detected in tumor tissues from D3 but the content was low. At D7, the TCR gene content in tumor tissues increased in the TCR-T group and the PD1(108)/CD28-TCR-T group, while the TCR gene content in tumor tissues of the PD1(108)/CD28-TIGIT/OX40-TCR-T group was lower than that of the other two groups. The peaks of expansion in tumor tissues of the TCR-T group and the PD1(108)/CD28-TCR-T group appeared at D10, and the extent of expansion was stronger in the PD1(108)/CD28-TCR-T group than in the TCR-T group, and then began to decline. In the tumor tissues of the PD1(108)/CD28-TIGIT/OX40-TCR-T group, the content of the TCR gene appeared to be greatly expanded from D10, and the peak of expansion appeared at D14, and the degree of expansion was stronger than that of the PD1(108)/CD28-TCR-T group, and the content remained at a higher level at D21-D35, and the content was the highest among the 3 groups. The enhanced receptor PD1(108)/CD28-TIGIT/OX40 had a stronger promotional effect on the proliferation and survival of TCR-T cells in mouse tumor tissues.

### Example 7. Effect of the enhanced receptor TIGIT/OX40 in human subjects

The effects of the TIGIT/OX40 enhanced receptor were further tested by clinical trials in human subjects. Specifically, the effects of T cells (c610) modified with dual enhanced receptors using a combination of the TIGIT/OX40 enhanced receptor and PD1(108)/CD28 were compared relative to T cells (c410) modified with PD1(108)/CD28 alone. This Example used PD1(108)/CD28-TCR-T cells and PD1(108)/CD28-TIGIT/OX40-TCR-T cells similar to those described in Examples 4-7.

### Procedure for c410 (PD1(108)/CD28-CD19 CAR-T) cells and c610 (PD1(108)/CD28-TIGIT/OX40-CD19 CAR-T) cells preparation

1. Day 0:
   (1) PD1+ T cell positively selecting:
      a. Took apheresis blood from patients, added anti-PD1-Biotin antibody (BioLegend, 329934), mix well and incubate away from light;
      b. Washed with buffer and centrifuged;
      c. Resuspended with buffer, add 20 µl anti-Biotin MicroBeads (Miltenyi Biotec, Germany, 170-076-709), mix well and incubate away from light;
      d. Washed with buffer, centrifuged, and resuspended the cells, while the column was rinsed with buffer;
      e. Removed the adsorption column after the cells have passed through the column, put it on a new centrifuge tube, added buffer in the column, quickly pushed out the liquid in the column with a piston, collected PD1+ cells and count them;
      f. According to the experimental needs, took the corresponding number of PD1+ cells for centrifugation, discarded the supernatant, and resuspended the cells with complete culture medium.
   (2) Added anti-CD3/CD28 magnetic beads to activate T cells:
      a. Took the appropriate number of Dynabeads CD3/CD28 (Thermo Fisher Scientific, 40203D) according to a certain ratio and placed them in a centrifuge tube;
      b. The magnetic beads were resuspended and washed using X-VIVO medium (Lonza, BEBP02-054Q), adsorbed on a magnetic rack for resting, and the supernatant was removed with a pipette after the beads were adsorbed to the tube wall;
      c. Cells and magnetic beads were resuspended using T_{SCM} medium (X-VIVO + 2.5% CTS^{™} Immune Cell SR (Thermo Fisher Scientific, A2596102) + 2 U/ml Memory Stem Cell Expansion Factor (Shanghai Novoprotein Scientific Inc., GMP-1647)) and transferred together into culture flasks, replenished with TSCM medium and adjust the cell density to 1.2×10⁶/ml;
      d. Placed the cells in 37°C, 5% CO2 incubator for incubation.
2. Day 1: Addition of lentivirus
   a. The time for lentiviral transfection was within 24 hours of cell activation;
   b. Based on the type of cells to be prepared, the type of lentivirus to be used (410 or 610; where 410 is PD1(108)/CD28-CD19 CAR and 610 is PD1(108)/CD28-TIGIT/OX40-CD19 CAR lentivirus) and the MOI were determined, and the volume of lentivirus added= Cells number × MOI / lentivirus titer, was calculated based on the number of cells;
   c. Took the corresponding volume of lentivirus from the -80°C refrigerator, inserted into the ice, and passed into the laboratory through the transfer window, the lentivirus was left to melt in the ice;
   d. The cells that need to be added with lentivirus were taken from the incubator, and the information of the cells that need to be added with lentivirus, patient name, cell name, and date of preparation are checked to confirm that there is no error;
   e. The thawed lentivirus was added to the cells, blown and mixed with a pipettor, and placed at 37°C, 5% incubator for culture.
3. Day 3: The cells were rehydrated and T_{SCM} medium was added according to the number of cells.
4. Day 5: Removal of magnetic beads
   a. In the biological safety cabinet, the corresponding volume of magnetic racks, centrifuge tubes were prepared (>20 ml: 50 ml centrifuge tubes; ≤20 ml: 15 ml centrifuge tubes; less than 1 ml: EP tubes), according to the volume of medium of the cells to be removed from the magnetic beads;
   b. Cells with magnetic beads were put through the magnetic rack three times, the beads were removed, and the cell suspension was collected;
   c. Washed the magnetic beads 2-3 times and collected the cell suspension again;
   d. Combined the cell suspensions obtained in b and c, recorded the total volume and counted the cells;
   e. leveled the weight of the tube, centrifuged and discarded the supernatant;
   f. Add complete medium (X-VIVO + 2.5% CTS^{™} immune cell SR (Thermo Fisher Scientific, A2596102) + 3000 IU/ml recombinant human interleukin 2 for injection (Beijing SL Pharmaceutical Co., Ltd., China State Drug Approval Number S19991010)), resuspend it and then transfer it to culture flasks, and according to the counting results, supplemented with complete medium to adjust the cell density, labeled with information;
   g. Put in 37°C, 5% CO2 incubator for culture.
5. Day 8: Cell counting, rehydration
6. Day 11: Cell counting, rehydration
7. Day 14:
   (1) Collection of finished product cells:
      a. The cells to be collected were taken from the incubator, the cell suspension was mixed with a pipetter, samples were taken for counting and calculating cell viability, and some samples were kept for flow cytometry;
      b. The cell suspension was transferred to a 200ml centrifuge tube/50ml centrifuge tube and centrifuged at 500g for 8min;
      c. After centrifugation, the supernatant was taken to the freezing tube, a total of 2 tubes (for mycoplasma, endotoxin, Gram's test);
      d. The supernatant was discarded, resuspended and washed with normal saline to 200 ml/50 ml, and centrifuged at 600 g for 10 min;
      e. Discarded the supernatant, resuspend the cells with a quantitative amount of normal saline, mixed well, took samples for counting and calculating cell viability, centrifuged, and discarded the supernatant;
      f. According to the number of cells, cells were resuspended with a quantitative amount of finished cell freezing medium product (Cryostor CS10, BioLife Solutions, 100-1061).
   (2) Cryostorage of cells:
      a. The cells added with freezing medium were quickly dispensed into freezing bags (the freezing bags need to be labeled with patient's name, number, cell details, freezing time and other information) and the freezing bags were placed into a programmed cooling box, with a -80°C refrigerator for freezing;
      b. Within 24 hours of being placed in the -80°C refrigerator, the freezing bags were transferred to liquid nitrogen for preservation;

Information such as the number of freezing bags was recorded.

### Inclusion Criteria

The study population enrolled in c410/c610 is adult patients with relapsed or refractory CD19-positive large B-cell lymphoma after prior second-line or higher systemic therapy, including: Diffuse large B-cell lymphoma (DLBCL) nonspecific (NOS), primary mediastinal large B-cell lymphoma (PMBCL), high-grade B-cell lymphoma, and DLBCL transformed from follicular lymphoma, follicular cell lymphoma, and mantle cell lymphoma.

The dose-increment group was divided into three groups of 1×10⁶/kg, 3.3×10⁶/kg, and 10×10⁶/kg, and the dose-expansion group was recommended as the cellular dose for the expansion trial, based on the safety and preliminary efficacy of the dose-increment trial.

The inclusion criteria mainly included: age between 18 and 70 years old, no gender restriction, ECOG score of 0 or 1, peripheral blood PD1-positive T cells as a percentage of total T cells of ≥18%, basic cardiopulmonary reserve, and no serious hematologic, hepatic or renal function abnormalities; the exclusion criteria mainly included: combination of active central nervous system lymphoma, active central nervous system disease; combination of malignant tumors and immunological system diseases; with severe cardiopulmonary functional impairment; presence of clinical emergencies; recently underwent major surgery and other unlisted clinical investigational drugs or treatments.

Autologous peripheral blood mononuclear cells were collected from subjects who passed the screening (PBMCs ≥ 3 × 10⁹), and PD-1 positive T lymphocytes were obtained by selecting, and the cells were used in the preparation of cell preparations.

Prior to cell reinfusion, subjects underwent a thorough baseline examination and lesion evaluation. c410/c610 cell reinfusion was given to subjects who received pretreatment chemotherapy (FC regimen, cyclophosphamide 250-300 mg/m² and fludarabine 25-30 mg/m² once daily for 3 days) on days D-5 to D-3, and c410/c610 cell infusion was given on day D0.

Subjects were followed up after the cell infusion, and the tolerability of c410 cells/c610 cells in the treatment of CD19-positive subjects with relapsed or refractory non-Hodgkin's lymphoma were evaluated, dose-limiting toxicity (DLT), safety and efficacy were observed.

### Results

Detailed data on the basic information and treatment outcomes of the subjects in the above clinical trials were shown in Table 4.

After c410/c610 cell treatment, all subjects were well tolerated without dose-limiting toxicity, and a CRS of up to grade 2.

The objective response rate (ORR) was 0% (0/1, 0/3) for one subject in the low-dose group (1×10⁶/kg) of c410 cells, and three subjects in the medium-dose group (3.3×10⁶/kg). A total of 6 cases were included in the high-dose group (10×10⁶/kg), with a complete remission rate (CR) of 50% (3/6) at D28, and CR of 33.3% (2/6) at W12 . The ORR was 83.3% (5/6) at D28, and ORR of 33.3% (2/6) at W12.

Compared to c410, the 2 subjects in the low-dose group (1 x 10⁶/kg) of c610 cells had a CR of 100% (2/2) at D28, which was significantly better than the results of treatment with c410 cells.

As seen in the results shown in Table 4 below, the efficacy of immune cells containing only the PD1(108)/CD28 single enhanced receptor (c410) was progressively enhanced with increasing dose (from 1E6/kg in the low-dose group, to 3.3E6/kg in the medium-dose group, to 1E7/kg in the high-dose group), but the risk of cytokine storm (CRS) was also increased.

In contrast, immune cells containing the PD1(108)/CD28-TIGIT/OX40 dual enhanced receptors (c610) had significantly better therapeutic efficacy and safety, with c610 at the low-dose (1E6/kg) being much more efficacious than the equivalent low-dose group of c410, and even significantly better than the high-dose group of c410, and with a lower risk of CRS than the various dose groups of c410. The results of this clinical trial illustrate the surprising therapeutic efficacy of the TIGIT/OX40 enhanced receptor of the present invention on tumors when combined with the PD1(108)/CD28 enhanced receptor.

**Table 4: Basic information and treatment outcomes of the subjects**

| Cell type (strengthen ed receptor) | Dose grouping (/kg) | Screen ing numbe r | Disease type and stage | CRS | Evaluati on at week 4 | Evaluation at week 12 | CR | ORR | DCR |
|---|---|---|---|---|---|---|---|---|---|
| c410 (PD1 streng thened receptor) | 1×10⁶ | S001 | B-lymphoblastic lymphoma stage IV | grade 1 | PD | - | 0%(0/1) | 0%(0/1) | 0%(0/1) |
| | 3.3×10⁶ | S002 | Mantle cell lymphoma stage IV | grade 1 | PD | - | 0%(0/3) | **D28** 33%(1/3) | **D28** 66.7%(2/3) |
| | | S003 | Diffuse large B-cell lymphoma stage IV | grade 1 | SD | Dropout | | | |
| | | | | | | | | **W12** 0%(0/3) | **W12** 0%(0/3) |
| | | S004 | DLBCL transformed from follicular lymphoma stage III | grade 1 | PR | PD | | | |
| | 10×10⁶ | S005 | Follicular lymphoma stage IV | grade 1 | CR | CR | **D28** *50%(3*/*6)* | **D28** 83.3%(5/6) | **D28** 83.3%(5/6) |
| | | S007 | Diffuse large B-cell lymphoma stage IV | grade 1 | PD | - | | | |
| | | S008 | Follicular Lymphoma stage III | grade 2 | PR | PD | | | |
| | | S009 | Diffuse large B-cell lymphoma stage IV | grade 2 | CR | PD | **W12** 33.3%(2/6) | **W12** 33.3%(2/6) | **W12** 50%(3/6) |
| | | S010 | Diffuse large B-cell lymphoma stage III | grade 2 | CR | CR | | | |
| | | S012 | Diffuse large B-cell lymphoma stage IV | grade 1 | PR | SD | | | |
| c610 (PD1-TIGI T dual strengthene d receptor) | 1×10⁶ | S002 | Diffuse large B-cell lymphoma stage IV | grade 1 | CR | CR | **D28** 100%(2/2) | **D28** 100%(2/2) | **D28** 100%(2/2) |
| | | S003 | Diffuse large B-cell lymphoma stage IV | None | CR | CR | **W12** 100%(212) | **W12** 100%(2/2) | **W12** 100%(2/2) |

### Informations of sequences

| SEQ ID No. | name | DN A/A A | sequences | note |
|---|---|---|---|---|
| 1 | TIGIT ECD | DN A | ATGCGGTGGTGCCTGCTGCTGATCTGGGCCCAGGGGCTGAGGCAGGCC CCCCTGGCCAGCGGCATGATGACAGGCACAATTGAGACAACAGGCAAC ATCAGCGCCGAGAAGGGCGGGAGCATCATCCTGCAGTGCCACCTGAGC AGCACCACCGCCCAGGTGACACAGGTGAACTGGGAGCAGCAGGACCA GCTGCTGGCCATCTGCAACGCCGATCTGGGCTGGCACATCAGCCCAAG CTTCAAGGATAGGGTGGCCCCCGGCCCCGGCCTGGGGCTGACCCTGCA GAGCCTGACCGTCAACGACACCGGGGAGTACTTCTGCATCTACCACAC ATACCCCGACGGCACCTACACCGGGCGGATCTTCCTGGAGGTGCTGGA GAGCAGCGTGGCCGAGCACGGCGCCAGATTCCAGATCCCC | WT |
| 2 | OX40 ICD | DN A | AGGCGGGATCAGCGGCTGCCCCCCGACGCCCACAAGCCCCCCGGCGGG GGCTCCTTCAGGACACCCATCCAGGAGGAGCAGGCCGATGCCCACAGC ACCCTGGCCAAGATC | WT |
| 3 | OX40 TM | DN A | GTGGCCGCCATCCTCGGGCTGGGGCTGGTCCTGGGGCTGCTGGGCCCCC TGGCCATCCTGCTGGCCCTGTACCTGCTG | WT |
| 4 | PD1(10 8) | DN A | ATGCAGATCCCACAGGCGCCCTGGCCAGTCGTCTGGGCGGTGCTACAA CTGGGCTGGCGGCCAGGATGGTTCTTAGACTCCCCAGACAGGCCCTGG AACCCCCCCACCTTCTCCCCAGCCCTGCTCGTGGTGACCGAAGGGGACA ACGCCACCTTCACCTGCAGCTTCTCCAACACATCGGAGAGCTTCGTGCT AAACTGGTACCGCATGAGCCCCAGCAACCAGACGGACAAGCTGGCCGC CTTCCCCGAGGACCGTATTAATAGGAGTCAGGACTGCCGCTTCCGTGTC ACACAACTGCCCAACGGGCGTGACTTCCACATGAGCGTGGTCAGGGCC CGGCGCAATGACAGCGGCACCTACCTCTGTGGGGCCATCTCCCTGTCTA CTCCGGTGTGGATCAAAGAGAGCCTGCGGGCAGAGCTCAGGGTGACAG AGAGAAGGGCAGAAGTGCCCACAGCCCAC | Nucleotid e sequence of a variant of PD1 |
| 5 | CD28 ICD | DN A | AGGAGTAAGAGGAGCAGGCTCCTGCACAGTGACTACATGAACATGACT CCCCGCCGCCCCGGGCCCACCCGCAAGCATTACCAGCCCTATGCCCCAC CACGCGACTTCGCAGCCTATCGCTCC | WT |
| 6 | CD28 TM | DN A | TGTCCAAGTCCCCTATTTCCCGGACCTTCTAAGCCCTTTTGGGTGCTGGT GGTGGTTGGTGGAGTCCTGGCTTGCTATAGCTTGCTAGTAACAGTGGCC TTTATTATTTTCTGGGTG | WT |
| 7 | AntiPD L1 scFv | DN A | ATGGCCTTACCAGTGACCGCCTTGCTCCTGCCGCTGGCCTTGCTGCTCC ACGCCGCCAGGCCGGACATCCAGATGACCCAGAGCCCTAGCAGCCTGA GCGCTAGCGTGGGAGATAGAGTGACCATCACCTGCAGGGCCTCTCAGG ATGTGTCTACAGCCGTGGCTTGGTACCAGCAGAAGCCAGGAAAGGCCC CCAAGCTGCTGATCTACAGCGCCTCTTTCCTGTACAGCGGCGTGCCTAG CAGATTCAGCGGCAGCGGAAGCGGCACCGATTTCACCCTGACCATCAG CAGCCTGCAGCCAGAGGACTTCGCCACCTACTACTGCCAGCAGTACCTG TACCACCCAGCCACCTTCGGACAGGGCACCAAGGTGGAGATCAAGGGT GGCGGTGGCTCGGGCGGTGGTGGGTCGGGTGGCGGCGGATCTGAGGTG CAGCTGGTGGAATCAGGAGGAGGACTGGTGCAGCCAGGAGGATCTCTG AGACTGTCTTGCGCCGCCAGCGGCTTTACATTCAGCGACTCTTGGATCC ATTGGGTGCGCCAGGCTCCAGGAAAAGGACTCGAGTGGGTGGCTTGGA TCAGCCCTTACGGCGGCAGCACCTACTACGCCGATAGCGTGAAGGGCA GGTTCACCATCAGCGCCGATACCAGCAAGAACACCGCCTACCTGCAGA TGAACAGCCTGAGGGCCGAGGATACCGCCGTGTACTATTGCGCCAGGA GGCATTGGCCAGGCGGATTTGACTATTGGGGCCAGGGAACACTGGTGA CCGTGTCCAGC | Sequence of anti-PDL1 monoclon al antibody scFv |
| 8 | PD1 ECD | DN A | ATGCAGATCCCACAGGCGCCCTGGCCAGTCGTCTGGGCGGTGCTACAA CTGGGCTGGCGGCCAGGATGGTTCTTAGACTCCCCAGACAGGCCCTGG AACCCCCCCACCTTCTCCCCAGCCCTGCTCGTGGTGACCGAAGGGGACA ACGCCACCTTCACCTGCAGCTTCTCCAACACATCGGAGAGCTTCGTGCT AAACTGGTACCGCATGAGCCCCAGCAACCAGACGGACAAGCTGGCCGC CTTCCCCGAGGACCGCAGCCAGCCCGGCCAGGACTGCCGCTTCCGTGTC ACACAACTGCCCAACGGGCGTGACTTCCACATGAGCGTGGTCAGGGCC CGGCGCAATGACAGCGGCACCTACCTCTGTGGGGCCATCTCCCTGGCCC CCAAGGCGCAGATCAAAGAGAGCCTGCGGGCAGAGCTCAGGGTGACA GAGAGAAGGGCAGAAGTGCCCACAGCCCAC | WT |
| 9 | TIGIT ECD | AA | MRWCLLLIWAQGLRQAPLASGMMTGTIETTGNISAEKGGSIILQCHLSSTT AQVTQVNWEQQDQLLAICNADLGWHISPSFKDRVAPGPGLGLTLQSLTVN DTGEYFCIYHTYPDGTYTGRIFLEVLESSVAEHGARFQIP | |
| 10 | OX40 ICD | AA | RRDQRLPPDAHKPPGGGSFRTPIQEEQADAHSTLAKI | |
| 11 | OX40 TM | AA | VAAILGLGLVLGLLGPLAILLALYLL | |
| 12 | PD(108) ECD | AA | MQIPQAPWPVVWAVLQLGWRPGWFLDSPDRPWNPPTFSPALLVVTEGDN ATFTCSFSNTSESFVLNWYRMSPSNQTDKLAAFPEDRINRSQDCRFRVTQL PNGRDFHMSVVRARRNDSGTYLCGAISLSTPVWIKESLRAELRVTERRAEV PTAH | |
| 13 | CD28 ICD | AA | RSKRSRLLHSDYMNMTPRRPGPTRKHYQPYAPPRDFAAYRS | |
| 14 | CD28 TM | AA | CPSPLFPGPSKPFWVLVVVGGVLACYSLLVTVAFIIFWV | |
| 15 | AntiPD L1 scFv | AA | MALPVTALLLPLALLLHAARPDIQMTQSPSSLSASVGDRVTITCRASQDVS TAVAWYQQKPGKAPKLLIYSASFLYSGVPSRFSGSGSGTDFTLTISSLQPED FATYYCQQYLYHPATFGQGTKVEIKGGGGSGGGGSGGGGSEVQLVESGG GLVQPGGSLRLSCAASGFTFSDSWIHWVRQAPGKGLEWVAWISPYGGSTY YADSVKGRFTISADTSKNTAYLQMNSLRAEDTAVYYCARRHWPGGFDYW GQGTLVTVSS | |
| 16 | PD1 ECD | AA | MQIPQAPWPVVWAVLQLGWRPGWFLDSPDRPWNPPTFSPALLVVTEGDN ATFTCSFSNTSESFVLNWYRMSPSNQTDKLAAFPEDRSQPGQDCRFRVTQL PNGRDFHMSVVRARRNDSGTYLCGAISLAPKAQIKESLRAELRVTERRAEV PTAH | |
| 17 | TCR | AA | METLLGLLILWLQLQWVSSKQEVTQIPAALSVPEGENLVLNCSFTDSAIYN LQWFRQDPGKGLTSLLLIQSSQREQTSGRLNASLDKSSGRSTLYIAASQPGD SATYLCAVRPLYGGSYIPTFGRGTSLIVHPYIQNPDPAVYQLRDSKSSDKSV CLFTDFDSQTNVSQSKDSDVYITDKTVLDMRSMDFKSNSAVAWSNKSDFA CANAFNNSIIPEDTFFPSPESSCDVKLVEKSFETDTNLNFQNLSVIGFRILLLK VAGFNLLMTLRLWSSQCTNYALLKLAGDVESNPGPMSIGLLCCAALSLLW AGPVNAGVTQTPKFQVLKTGQSMTLQCAQDMNHEYMSWYRQDPGMGL RLIHYSVGAGITDQGEVPNGYNVSRSTTEDFPLRLLSAAPSQTSVYFCASSY VGNTGELFFGEGSRLTVLEDLKNVFPPEVAVFEPSEAEISHTQKATLVCLAT GFFPDHVELSWWVNGKEVHSGVSTDPQPLKEQPALNDSRYCLSSRLRVSA TFWQNPRNHFRCQVQFYGLSENDEWTQDRAKPVTQIVSAEAWGRADCGF TSVSYQQGVLSATILYEILLGKATLYAVLVSALVLMAMVKRKDF | |
| 18 | TCR | DN A | ATGGAGACCCTCTTGGGCCTGCTTATCCTTTGGCTGCAGCTGCAATGGG TGAGCAGCAAACAGGAGGTGACGCAGATTCCTGCAGCTCTGAGTGTCC CAGAAGGAGAAAACTTGGTTCTCAACTGCAGTTTCACTGATAGCGCTAT TTACAACCTCCAGTGGTTTAGGCAGGACCCTGGGAAAGGTCTCACATCT CTGTTGCTTATTCAGTCAAGTCAGAGAGAGCAAACAAGTGGAAGACTT AATGCCTCGCTGGATAAATCATCAGGACGTAGTACTTTATACATTGCAG CTTCTCAGCCTGGTGACTCAGCCACCTACCTCTGTGCTGTGAGGCCCTT ATACGGAGGAAGCTACATACCTACATTTGGAAGAGGAACCAGCCTTAT TGTTCATCCGTACATCCAGAACCCTGACCCTGCCGTGTACCAGCTGAGA GACTCTAAATCCAGTGACAAGTCTGTCTGCCTATTCACCGATTTTGATT CTCAAACAAATGTGTCACAAAGTAAGGATTCTGATGTGTATATCACAG ACAAAACTGTGCTAGACATGAGGTCTATGGACTTCAAGAGCAACAGTG CTGTGGCCTGGAGCAACAAATCTGACTTTGCATGTGCAAACGCCTTCAA CAACAGCATTATTCCAGAAGACACCTTCTTCCCCAGCCCAGAAAGTTCC TGTGATGTCAAGCTGGTCGAGAAAAGCTTTGAAACAGATACGAACCTA AACTTTCAAAACCTGTCAGTGATTGGGTTCCGAATCCTCCTCCTGAAAG TGGCCGGGTTTAATCTGCTCATGACGCTGCGGCTGTGGTCCAGCCAATG TACTAACTACGCTTTGTTGAAACTCGCTGGCGATGTTGAAAGTAACCCC GGTCCTATGAGCATCGGCCTCCTGTGCTGTGCAGCCTTGTCTCTCCTGTG GGCAGGTCCAGTGAATGCTGGTGTCACTCAGACCCCAAAATTCCAGGT CCTGAAGACAGGACAGAGCATGACACTGCAGTGTGCCCAGGATATGAA CCATGAATACATGTCCTGGTATCGACAAGACCCAGGCATGGGGCTGAG GCTGATTCATTACTCAGTTGGTGCTGGTATCACTGACCAAGGAGAAGTC CCCAATGGCTACAATGTCTCCAGATCAACCACAGAGGATTTCCCGCTCA GGCTGCTGTCGGCTGCTCCCTCCCAGACATCTGTGTACTTCTGTGCCAG CAGTTACGTCGGGAACACCGGGGAGCTGTTTTTTGGAGAAGGCTCTAG GCTGACCGTACTGGAGGACCTGAAGAACGTGTTCCCACCCGAGGTCGC TGTGTTTGAGCCATCAGAAGCAGAGATCTCCCACACCCAAAAGGCCAC ACTGGTGTGCCTGGCCACAGGCTTCTTCCCTGACCACGTGGAGCTGAGC TGGTGGGTGAATGGGAAGGAGGTGCACAGTGGGGTCAGCACGGACCCG CAGCCCCTCAAGGAGCAGCCCGCCCTCAATGACTCCAGATACTGCCTG AGCAGCCGCCTGAGGGTCTCGGCCACCTTCTGGCAGAACCCCCGCAAC CACTTCCGCTGTCAAGTCCAGTTCTACGGGCTCTCGGAGAATGACGAGT GGACCCAGGATAGGGCCAAACCCGTCACCCAGATCGTCAGCGCCGAGG CCTGGGGTAGAGCAGACTGTGGCTTTACCTCGGTGTCCTACCAGCAAGG GGTCCTGTCTGCCACCATCCTCTATGAGATCCTGCTAGGGAAGGCCACC CTGTATGCTGTGCTGGTCAGCGCCCTTGTGTTGATGGCCATGGTCAAGA GAAAGGATTTCTAA | |
| 19 | CD19 | AA | MGWSCIILFLVATATGVHSAIQLTQSPSSLSASVGDRVTITCRASQGISSALA | |
| | CAR | | WYQQKPGKAPKLLIYDASSLESGVPSRFSGSGSGTDFTLTISSLQPEDFATY YCQQFNSYPYTFGQGTKLEIKGGGGSGGGGSGGGGSEVQLVQSGAEVKKP GESLKISCKGSGYSFSSSWIGWVRQMPGKGLEWMGIIYPDDSDTRYSPSFQ GQVTISADKSIRTAYLQWSSLKASDTAMYYCARHVTMIWGVIIDFWGQGT LVTVSSAAATTTPAPRPPTPAPTIASQPLSLRPEACRPAAGGAVHTRGLDFA CDIYIWAPLAGTCGVLLLSLVITLYCKRGRKKLLYIFKQPFMRPVQTTQEE DGCSCRFPEEEEGGCELRVKFSRSADAPAYKQGQNQLYNELNLGRREEYD VLDKRRGRDPEMGGKPRRKNPQEGLYNELQKDKMAEAYSEIGMKGERRR GKGHDGLYQGLSTATKDTYDALHMQALPPR | |
| 20 | CD19 CAR | DN A | ATGGGCTGGTCTTGCATCATCCTGTTCCTCGTGGCCACCGCCACCGGCG TCCACAGCGCCATCCAGCTCACCCAGAGCCCCTCGAGCTTGAGTGCCTC GGTGGGAGACCGGGTCACTATCACCTGCCGAGCCAGTCAGGGCATCTC CTCCGCCCTTGCCTGGTACCAGCAGAAGCCCGGGAAGGCCCCCAAGCT GCTGATCTACGACGCTAGTAGTCTGGAGAGTGGCGTGCCTTCGCGCTTC TCGGGCAGTGGGAGTGGCACCGACTTCACCTTGACCATCTCCAGTCTAC AGCCGGAAGATTTCGCGACCTACTACTGTCAGCAATTCAACTCTTATCC ATACACTTTCGGCCAGGGGACAAAGCTGGAGATCAAGGGCGGGGGCGG GAGTGGCGGCGGAGGGTCCGGAGGCGGGGGCTCCGAGGTGCAACTAGT CCAGAGCGGAGCCGAGGTGAAGAAGCCCGGGGAGAGTCTAAAGATCT CTTGCAAGGGCTCCGGTTACTCCTTCTCGAGTTCCTGGATCGGGTGGGT GCGACAGATGCCGGGCAAGGGCCTGGAGTGGATGGGCATTATCTACCC CGACGACTCCGATACCCGTTATAGTCCATCGTTCCAGGGACAGGTGACC ATTTCCGCCGACAAGTCTATCAGAACCGCCTATCTGCAGTGGTCCAGTC TGAAGGCCTCTGACACTGCCATGTATTATTGCGCCAGGCACGTTACGAT GATCTGGGGGGTGATCATCGACTTCTGGGGCCAGGGCACACTCGTAAC CGTCAGTTCTGCGGCCGCAACCACGACGCCAGCGCCGCGACCACCAAC ACCGGCGCCCACCATCGCGTCGCAGCCCCTGTCCCTGCGCCCAGAGGC GTGCCGGCCAGCGGCGGGGGGCGCAGTGCACACGAGGGGGCTGGACTT CGCCTGTGATATCTACATCTGGGCGCCCTTGGCCGGGACTTGTGGGGTC CTTCTCCTGTCACTGGTTATCACCCTTTACTGCAAACGGGGCAGAAAGA AACTCCTGTATATATTCAAACAACCATTTATGAGACCAGTACAAACTAC TCAAGAGGAAGATGGCTGTAGCTGCCGATTTCCAGAAGAAGAAGAAGG AGGATGTGAACTGAGAGTGAAGTTCAGCAGGAGCGCAGACGCCCCCGC GTACAAGCAGGGCCAGAACCAGCTCTATAACGAGCTCAATCTAGGACG AAGAGAGGAGTACGACGTTTTGGACAAGAGACGTGGCCGGGACCCTGA GATGGGGGGAAAGCCGAGAAGGAAGAACCCTCAGGAAGGCCTGTACA ATGAACTGCAGAAAGATAAGATGGCGGAGGCCTACAGTGAGATTGGGA TGAAAGGCGAGCGCCGGAGGGGCAAGGGGCACGATGGCCTTTACCAG GGTCTCAGTACAGCCACCAAGGACACCTACGACGCCCTTCACATGCAG GCCCTGCCCCCTCGCTAA | |
| 21 | T2A | AA | EGRGSLLTCGDVEENPGP | |
| 22 | T2A | DN A | GAGGGCAGAGGAAGTCTTCTAACATGCGGTGACGTGGAGGAGAATCCC GGCCCT | |

## Claims

1. A enhanced receptor comprising an extracellular domain (ECD), a transmembrane domain (TM), and an intracellular domain (ICD), wherein the extracellular domain is derived from an extracellular domain of TIGIT, and the intracellular domain is derived from an intracellular domain of OX40.

2. The enhanced receptor according to claim 1, wherein the extracellular domain is the extracellular domain of human wild-type TIGIT, or a fragment or variant of the extracellular domain of human wild-type TIGIT that is capable of binding to a TIGIT ligand.

3. The enhanced receptor according to claim 2, wherein the extracellular domain has an amino acid sequence as shown in SEQ ID NO: 9.

4. The enhanced receptor according to any one of claims 1 or 3, wherein the intracellular domain is the intracellular domain of human OX40, or a fragment or variant of the intracellular domain of human OX40.

5. The enhanced receptor according to claim 4, wherein the intracellular domain has an amino acid sequence as shown in SEQ ID NO: 10.

6. The enhanced receptor of any one of claims 1 to 5, wherein the transmembrane domain is derived from the transmembrane domain of TIGIT or OX40.

7. The enhanced receptor according to claim 6, wherein the transmembrane domain is derived from the transmembrane domain of OX40 and has an amino acid sequence as shown in SEQ ID NO: 11.

8. An isolated nucleic acid molecule comprising a nucleotide sequence encoding the enhanced receptor according to any one of claims 1-7.

9. The isolated nucleic acid molecule according to claim 8, comprising one or more of the following nucleotide sequences, preferably comprising (1) and (2), more preferably comprising (1) to (3):
(1) a nucleotide sequence as shown in SEQ ID NO: 1 encoding the extracellular domain derived from TIGIT;
(2) a nucleotide sequence as shown in SEQ ID NO: 2 encoding the intracellular domain derived from OX40; and
(3) a nucleotide sequence as shown in SEQ ID NO: 3 encoding the transmembrane domain derived from OX40.

10. A recombinant expression vector comprising the isolated nucleic acid molecule according to claims 8 or 9.

11. A host cell comprising the isolated nucleic acid molecule according to claims 8 or 9, or the recombinant expression vector according to claim 10.

12. An immune cell which is modified to express the enhanced receptor according to any one of claims 1 to 7.

13. The immune cell according to claim 12, the modification of the immune cell is carried out by introducing into the immune cell the isolated nucleic acid molecule according to claim 8 or 9, or the recombinant expression vector according to claim 10.

14. The immune cell according to claim 12 or 13, which is modified to further express a second enhanced receptor in addition to the enhanced receptor of claims 1-7, wherein the second enhanced receptor comprises a second extracellular domain, a second transmembrane domain, and a second intracellular domain, wherein
the second extracellular domain is capable of specifically binding to a ligand for a T cell immune checkpoint molecule different from TIGIT, wherein the second intracellular domain is derived from a co-stimulatory molecule that mediates immune cell activation signals.

15. The immune cell according to claim 14, wherein the extracellular domain of the second enhanced receptor is capable of specifically binding to PD-L1.

16. An immune cell according to claim 14 or 15, wherein the intracellular domain of the second enhanced receptor is derived from CD28.

17. The immune cell according to claim 15 or 16, wherein the second extracellular domain is the extracellular domain of PD-1, or a variant or fragment of the extracellular domain of PD-1 that is capable of binding to PD-L1.

18. The immune cell according to claim 15 or 16, wherein the second extracellular domain is an antibody that specifically binds to PD-L1, such as a single chain antibody (scFv) that specifically binds to PD-L1.

19. The immune cell according to claim 17, wherein the second extracellular domain has an amino acid sequence as shown in SEQ ID NO: 12 or SEQ ID NO: 16.

20. The immune cell according to any one of claims 12 to 19, which is modified to express a chimeric antigen receptor, wherein the CAR comprises:
(a) an antigen-interacting domain that is capable of binding to a B cell surface protein;
(b) a transmembrane domain; and
(c) an intracellular signaling domain.

21. The immune cell according to any one of claims 12 to 20, prior to being modified, the immune cell is isolated from peripheral blood mononuclear cells(PBMCs).

22. The immune cell according to claim 21, prior to being modified, the immune cell is obtained from PBMCs, and having undergone positive selection for PD-1.

23. An isolated nucleic acid molecule comprising the nucleotide sequence encoding the enhanced receptor according to any one of claims 1-7, and a nucleotide sequence encoding a second enhanced receptor, wherein
the second enhanced receptor comprises a second extracellular domain, a second transmembrane domain, and a second intracellular domain, wherein the second extracellular domain is capable of specifically binding to a ligand for a T cell immune checkpoint molecule different from TIGIT, wherein the second intracellular domain is derived from a co-stimulatory molecule that mediates immune cell activation signals.

24. The isolated nucleic acid molecule according to claim 23, wherein the extracellular domain of the second enhanced receptor is capable of specifically binding to PD-L1.

25. The isolated nucleic acid molecule according to claim 23 or 24, wherein the intracellular domain of the second enhanced receptor is derived from CD28.

26. The isolated nucleic acid molecule according to claim 24 or 25, wherein the second extracellular domain is the extracellular domain of PD-1, or a variant or fragment of the extracellular domain of PD-1 that is capable of binding to PD-L1.

27. The isolated nucleic acid molecule according to claim 24 or 25, wherein the second extracellular domain is an antibody that specifically binds to PD-L1, such as a single chain antibody (scFv) that specifically binds to PD-L1.

28. The isolated nucleic acid molecule according to claim 24 or 25, wherein the second extracellular domain has an amino acid sequence as shown in SEQ ID NO: 12 or SEQ ID NO: 16.

29. The isolated nucleic acid molecule according to claim 28, wherein the nucleotide sequence encoding the second extracellular domain has a nucleotide sequence as shown in SEQ ID NO: 4 or SEQ ID NO: 8.

30. The use of the immune cell according to any one of claims 12 to 22, in the preparation of a drug for the treatment of a disease.

31. The use according to claim 30, wherein the disease is a tumor.
